# EUROPEAN PATENT APPLICATION

(11) **EP 4 635 517 A1**
(43) Date of publication of application: **22.10.2025**
(21) Application number: 25170443.3
(22) Date of filing: 14.04.2025
(51) Int. Cl.: A61K 47/68, A61P 35/00

(54) **METAL-BASED ANTIBODY-DRUG CONJUGATES AND METHODS OF MAKING AND USING THEREOF**

(30) Priority: 19.04.2024 US 202463636226 P
(71) Applicant: Laboratory for Synthetic Chemistry and Chemical Biology Limited, Hong Kong Shatin (HK)
(72) Inventor: CHE, Chi Ming, Hong Kong (HK); CHU, Chun Ho, Hong Kong (HK)
(74) Representative: HGF

(57) **Abstract**

Metal-based antibody-drug conjugates are described herein. Methods of making and using thereof are also described. The conjugates, and pharmaceutical compositions thereof, can be used in anticancer therapies.

## Description

### CROSS-REFERENCED TO RELATED APPLICATIONS

This application claims the benefit of and priority to U.S. Provisional Application No. 63/636,226, filed April 19, 2024, which is hereby incorporated herein by reference in its entirety.

### FIELD OF THE INVENTION

This invention is generally in the field of methods of metal-based antibody-drug conjugates.

### BACKGROUND OF THE INVENTION

Cancer is the leading cause of death worldwide, accounting for nearly 10 million deaths in 2020. Chemotherapy is one of the major treatment modalities, however, the currently used cytotoxic agents cause serious systemic toxicity due to the nonselective mechanism of action, resulting in a narrow therapeutic window and undesirable off-target and dose-dependent toxicity.

Attempts to address these problems have included conjugation of tumor-targeting antibodies to deliver cytotoxic drugs to tumor sites while sparing normal tissues. Thus, antibody-drug conjugates have been developed in recent years. They are typically formed by conjugating a monoclonal antibody targeting a tumor-specific antigen with cytotoxic drugs via chemical linkers. To date, only about fifteen antibody-drug conjugates have been approved globally. However, development of severe side effects and drug resistance remain an issue.

Therefore, there remains a need and demand for exploring other cytotoxic payloads with multifaceted modes of action which address the aforementioned issues.

Therefore, it is the object of the present invention to provide novel antibody-drug conjugates.

It is a further object of the present invention to provide pharmaceutical compositions including such antibody-drug conjugates.

It is yet a further object of the present invention to provide methods of using such pharmaceutical compositions.

It is still a further object of the present invention to provide methods of making such antibody-drug conjugates.

### SUMMARY OF THE INVENTION

Described herein are metal-based antibody-drug conjugates (ADCs), as well as methods of making and using thereof. In particular, an ADC composed of a metallodrug gold(III) porphyrin and an monoclonal antibody are described, which can be used for treating cancer in subjects in need thereof.

In some non-limiting instances, a metal-based antibody-drug conjugate includes:
a gold(III) porphyrin;
a linker group; and
an antibody;
where the linker group covalently links the metalloporphyrin and the antibody.

The gold(III) porphyrin acts as a payload in the ADCs described. In some instances, the metal-based antibody-drug conjugate has a chemical structure according to Formula (I): 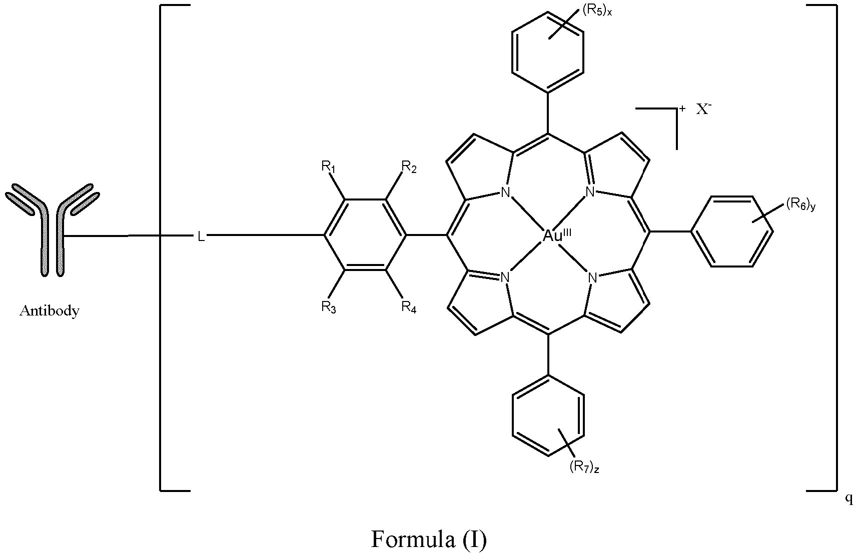
where L is the linker group;
where R₁, R₂, R₃, and R₄ are each independently selected from a hydrogen; halogen group (i.e., -F, -Cl, -Br, -I); a C₂-C₅ alkyl group (linear or branched), such as a methyl, ethyl, propyl, butyl, or pentyl group; alkenyl group; alkynyl group; cycloalkyl group; cycloalkenyl group; cycloalkynyl group; a hydroxyl group; an alkoxy group, such as methoxy, ethoxy, propoxy, or butoxy; an aryl group (i.e., a phenyl group); a heteroaryl group; a benzyl group; an acyl group; an ester group; a carbonyl group; a carboxylate group; an amino group (primary, secondary, or tertiary); an amide group; and a nitro group;
where R₅, R₆, and R₇ are optional substituents and are each independently selected from a halogen group (i.e., -F, -Cl, -Br, -I); a C₂-C₅ alkyl group (linear or branched), such as a methyl, ethyl, propyl, butyl, or pentyl group; alkenyl group; alkynyl group; cycloalkyl group; cycloalkenyl group; cycloalkynyl group; a hydroxyl group; an alkoxy group, such as methoxy, ethoxy, propoxy, or butoxy; an aryl group (i.e., a phenyl group); a heteroaryl group; a benzyl group; an acyl group; an ester group; a carbonyl group; a carboxylate group; an amino group (primary, secondary, or tertiary); an amide group; and a nitro group, ; and x, y, and z are integer values independently selected from 0, 1, 2, 3, 4, and 5;

wherein X⁻ is a pharmaceutically acceptable counterion; and
wherein q is a value in a range of about 1 to 8, or individual values or sub-ranges contained within.

In some particular instances, the metal-based antibody-drug conjugate has the following structures:

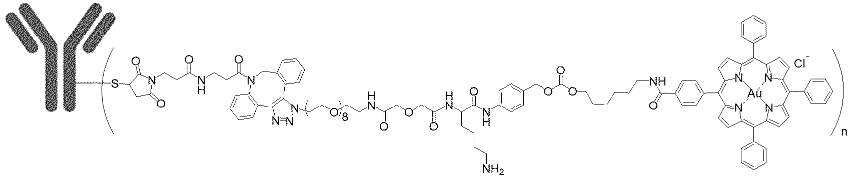

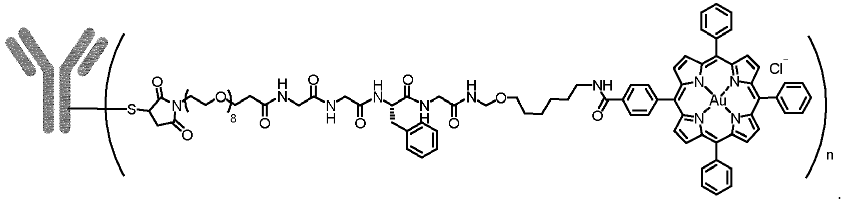

In some instances, the metal-based antibody-drug conjugates (ADCs) described herein, forms part of a pharmaceutical composition. The pharmaceutical composition includes an effective amount of the ADC and optionally one or more pharmaceutically acceptable carriers and/or excipients.

In one non-limiting instance, a pharmaceutical composition includes:
a plurality of the metal-based antibody-drug conjugates described above;
one or more pharmaceutically acceptable carriers; and
optionally one or more pharmaceutically acceptable excipients.

The metal-based antibody-drug conjugates described herein can be synthesized according to the methods described below. In one non-limiting instances, a method of making a metal-based antibody-drug conjugate includes the steps of:
(a) forming or providing a gold(III) porphyrin including a linker group; wherein the linker group comprises an end-group known as an antibody attachment moiety, such as a thiol-reactive Michael acceptor group and amine-reactive N-Hydroxysuccinimide group; and/or the linker group comprises a spacer, and/or the linker group comprises a cleavable bond; and
(b) conjugating the gold(III) porphyrin to an antibody, such as a monoclonal antibody, to form the metal-based antibody-drug conjugate;
   wherein the monoclonal antibody comprises one or more thiol groups and at least one of the thiol groups undergoes a Michael addition with the Michael acceptor group of the linker group; or the monoclonal antibody comprises one or more amino groups and at least one of the amino groups undergoes a condensation reaction with the N-hydroxysuccinimide of the linker group.

The disclosed ADCs can be used to treat cancers, such as cancers where the cells overexpress tumor-associated receptors, such as TROP2, HER2, Nectin4, folate receptor alpha, and EGFR receptors. The ADC is typically administered in a pharmaceutical composition to a subject in need thereof. In one non-limiting example, a method of targeted cancer therapy includes the steps of:
(i) administering a pharmaceutical composition containing a plurality of the ADCs described herein to a subject in need thereof.

In some instances, the subject has a cancer wherein the cancer cells overexpress tumor-associated receptors, such as TROP2, HER2, Nectin4, folate receptor alpha, and EGFR receptors. In some instances, the cancer is selected from breast, lung, liver, kidney, colorectal (such as colon), gastric, ovarian, and pancreatic cancer.

### BRIEF DESCRIPTION OF THE DRAWINGS

Non-limiting embodiments are described by way of example with reference to the accompanying Figure.
**Figures 1A-1C** shows an SDS-PAGE analysis of the light chain and heavy chain fragments of Sacituzumab (1A), Cetuximab (1B), and Trastuzumab (1C) and the corresponding ADC-Au.
**Figure 2A-2B** shows graphs of the UV-Vis absorption spectra of the monoclonal antibody (Sacituzumab (2A) and Trastuzumab (2B)), the corresponding ADC-Au, and Linker-AuOH.
**Figures 3A-3C** show the deconvoluted mass spectra of light chain and heavy chain fragments of Sacituzumab (3A), Cetuximab (3B), and Trastuzumab (3C) before and after conjugation with gold porphyrin.
**Figures 4A-4E****,** show graphs of the binding activity analysis of Sacituzumab, the corresponding ADC-Au, and IgG control with TROP2-overexpressed cancer cells, including HCC827, Calu3, BxPC3, SW1990, and the TROP2-negative non-cancerous HEK293 cells.
**Figure 4F** shows a bar graph of the quantified mean fluorescence intensity (MFI) of ADC-Au, Sacituzumab, and IgG control binding to the HCC827, Calu3, BxPC3, SW1990, and HEK293 cells.
**Figure 5** shows the curves of tumor volume of HCC827 xenograft mice treated with AuOH, Sacituzumab, ADC-Au, and vehicle control.
**Figures 6A** **and** **6B** show graphs of the binding activity analysis of Cetuximab, the corresponding ADC-Au, and IgG control with EGFR-overexpressed HCT119 and HT29 cells.
**Figure 6C** shows a bar graph of the quantified mean fluorescence intensity of Cetuximab, the corresponding ADC-Au, and IgG control binding to the HCT119 and HT29 cells.
**Figure 7A and 7B** show the image and weight of the tumor after 12 days of treatment.
**Figure 7C** shows the tumor growth curves of HT29 tumor-bearing mice being treated with Cetuximab, ADC-Au, and AuOH and the vehicle control.
**Figure 7D** shows the body weight of the mice during 12 days.
**Figure 8** shows the binding of Trastuzumab and ADC-Au to HER2 protein by ELISA.
**Figures 9A and 9B** show graphs of the binding activity analysis of Trastuzumab, the corresponding ADC-Au, and IgG control with HER2-overexpressed NCI-N87 cells and HER2-low MDA-MB-231 cells.
**Figure 9C** shows a bar graph of the quantified mean fluorescence intensity of Trastuzumab, the corresponding ADC-Au, and IgG control binding to the NCI-N87 and MDA-MB-231 cells.
**Figures 10A-C**show internalization analysis of ADC-Au and Trastuzumab with NCI-N87 cells.
**Figures 11A and 11B** show cell viability curves of NCI-N87 and MDA-MB-231 cells treated with different concentrations of Trastuzumab, the corresponding ADC-Au, and AuOH.
**Figure 12** shows western blot assay of pAkt and Akt in the NCI-N87 cells treated with Trastuzumab and the corresponding ADC-Au for 24 hours.
**Figure 13A** shows the image of the tumor after 18 days of treatment.
**Figures 13B and 13C** show the tumor growth curves and the body weight of NCI-N87 tumor-bearing mice being treated with Trastuzumab, ADC-Au, and AuOH and the vehicle control.

### DETAILED DESCRIPTION OF THE INVENTION

Metal-based antibody-drug conjugates (ADCs), as well as methods of making and using thereof, are described herein. In particular, an ADC composed of a metallodrug gold(III) porphyrin and an monoclonal antibody, such as TROP2-targeted Sacituzumab, EGFR-targeted Cetuximab, and HER2-targeted Trastuzumab, is described, which can be used for treating cancer in subjects in need thereof.

### I. Definitions

It is to be understood that the disclosed compounds, compositions, and methods are not limited to specific synthetic methods, specific analytical techniques, or to particular reagents unless otherwise specified, and, as such, may vary. It is also to be understood that the terminology used herein is for the purpose of describing particular forms and embodiments only and is not intended to be limiting.

The term "subject," as used herein, refers to a mammal, such as a human.

The term "effective amount" or "therapeutically effective amount" refers to the amount of a compound or agent which is able to treat one or more symptoms of a disease or disorder, reverse the progression of one or more symptoms of a disease or disorder, halt the progression of one or more symptoms of a disease or disorder, or prevent the occurrence of one or more symptoms of a disease or disorder in a subject to whom the formulation is administered, for example, as compared to a matched subject not receiving the compound. The actual effective amounts of a compound can vary according to the specific compound or combination thereof being utilized, the particular composition formulated, the mode of administration, and the age, weight, condition of the individual, and severity of the symptoms or condition being treated.

The term "pharmaceutically acceptable" refers to compositions or other materials and/or dosage forms which are, within the scope of sound medical judgment, suitable for use in contact with the tissues of human beings and animals without excessive toxicity, irritation, allergic response, or other problem or complication, commensurate with a reasonable benefit/risk ratio. The phrase "pharmaceutically acceptable carrier" refers to pharmaceutically acceptable materials, compositions or vehicles, such as a liquid or solid filler, diluent, solvent or encapsulating material involved in carrying or transporting any subject composition, from one organ, or portion of the body, to another organ, or portion of the body. Each carrier must be "acceptable" in the sense of being compatible with the other ingredients of a subject composition and not injurious to the subject to which it is administered.

The terms "inhibit" or "reduce" in the context of inhibition, mean to reduce or decrease in activity and quantity. This can be a complete inhibition or reduction in activity or quantity, or a partial inhibition or reduction. Inhibition or reduction can be compared to a control or to a standard level. Inhibition can be measured as a % value, e.g., from 1% up to 100%, such as 5%, 10, 25, 50, 75, 80, 85, 90, 95, 99, or 100%. For example, pharmaceutical compositions including one or more types of active agents may inhibit or reduce one or more markers of a disease or disorder, such as a cancer, in a subject by about 10%, 20%, 30%, 40%, 50%, 75%, 85%, 90%, 95%, or 99% from the activity and/or quantity of the same marker in subjects that did not receive or were not treated with the compositions.

The terms "treating" in the context of a disease or disorder means to ameliorate, reduce or otherwise stop a disease, disorder, or condition from occurring or progressing in an animal which may be predisposed to the disease, disorder and/or condition but has not yet been diagnosed as having it; inhibiting the disease, disorder or condition, e.g., impeding its progress; and relieving the disease, disorder, or condition, e.g., causing regression of the disease, disorder and/or condition. Treating the disease or condition includes ameliorating at least one symptom of the particular disease or condition, even if the underlying pathophysiology is not affected, such as treating the pain of a subject by administration of an analgesic agent even though such agent does not treat the cause of the pain. Desirable effects of treatment include decreasing the rate of disease progression, ameliorating, or palliating the disease state, and remission or improved prognosis. For example, a subject is "treated" if one or more symptoms associated with a cancer, such as cancer where the cancer cells overexpress TROP2 receptor, is mitigated or eliminated, including, but not limited to, increasing the quality of life of those suffering from the cancer, decreasing the dose of other medications required to treat the cancer, delaying the progression of the cancer, and/or prolonging survival of subject(s).

The terms "pharmaceutically acceptable ion," "pharmaceutically acceptable anion," "pharmaceutically acceptable cation," "pharmaceutically acceptable counter-ion," "pharmaceutically acceptable counter-anion," "pharmaceutically acceptable counter-cation," means an ion, anion, cation, counter-ion, counter-anion, or counter-cation, respectively, approved or accepted by a regulatory agency of the Federal or a state government or listed in the US. Pharmacopoeia or other generally recognized pharmacopoeia for use in animals, mammals, and more particularly in humans.

The phrase "counterion" refers to an ion associated with an oppositely charged conjugate or complex. Examples of counter-ions include counter-anions and counter-cations.

The phrase "counter-anion" refers to an anion associated with a positively charged conjugate or complex (e.g., a charged gold (III) complex). Non-limiting examples of counter-anions include halogens such as fluoride, chloride, bromide, and iodide, sulfate, phosphate, trifluoromethanesulfonate, acetate, nitrate, perchlorate, acetylacetonate, hexafluoroacetylacetonate and hexafluorophosphate.

The term "linker group" refers to the component connecting an antibody and a payload. It can include several components, such as one or more PEG units, spacer, and an antibody-attachment moiety that can conjugate to an antibody. Based on the drug release mechanism, the linker group can be classified as cleavable and non-cleavable in type.

The term "cleavable linkers" refers to linker groups which can be cleaved within target cells and release drug payloads. Examples include, without limitation, acid-sensitive linkers (such as hydrazones, carbonates, and acetals), which can be hydrolyzed in weakly acidic endosomes and lysosomes; glutathione-sensitive disulfide linkers, which can be cleaved under high intracellular glutathione concentrations in cancer cells; and enzymatically labile peptide linkers (such as dipeptide Valine-Citrulline, Valine-Alanine, Phenylalanine-Lysine, and Glycine-Glycine-Phenylalanine-Glycine), which can be effectively cleaved by an endolytic protease, such as Cathepsin B.

The term "non-cleavable linkers" refers to linker groups that function by releasing payloads upon lysosomal degradation of the whole antibody-drug conjugate (ADC). Examples include, without limitation, SMCC (N-succinimidyl 4-(maleimidomethyl)cyclohexane-1-carboxylate) and EMCS (N-ε-maleimidocaproyloxy-succinimide ester). The release component consists of the payload, linker, and the amino acid residue of the antibody that connects to the linker (such as lysine and cysteine).

An "aryl radical" or "aryl group" is understood to mean a radical containing a structure made up of 6 to 30 carbon atoms, 6 to 18 carbon atoms, which is formed from one aromatic ring or a plurality of fused aromatic rings. Exemplary aryl radicals are, without limitation, phenyl, naphthyl, anthracenyl, or phenanthrenyl. Aryl radicals may be unsubstituted, where all carbon atoms which are substitutable bear hydrogen atoms. Alternatively, they may be substituted at one, greater than one, or at all substitutable positions therein. Suitable exemplary substituents include, without limitation, alkyl radicals, such as alkyl radicals having 1 to 8 carbon atoms, which may be selected from methyl, ethyl, i-propyl or t-butyl, aryl radicals (such as C₆-aryl radicals, which may be substituted or unsubstituted), heteroaryl radicals (which may comprise at least one nitrogen atom, such as pyridyl radicals), alkenyl radicals (which may comprise one double bond and 1 to 8 carbon atoms), or groups with electron donating or electron accepting ability. Groups with electron donating ability are understood to mean groups which have a positive inductive (+I) and/or positive mesomeric (+M) effect, and groups with electron accepting ability are understood to mean groups which have a negative inductive (-I) and/or negative mesomeric (-M) effect. Suitable groups with donor or acceptor action are halogen radicals, such as F, Cl, Br, alkoxy radicals, aryloxy radicals, carbonyl radicals, ester radicals, amine radicals, amide radicals, CH₂F groups, CHF₂ groups, CF₃ groups, CN groups, thio groups, or SCN groups.

A "heteroaryl radical" or "heteroaryl group" is understood to mean radicals which differ from the aryl radicals described above in that at least one carbon atom in the structure making up the aryl radical is otherwise replaced by at least one heteroatom. Heteroatoms may have hydrogen substituents and/or any permissible substituents of organic compounds in order to satisfy the valences of the heteroatoms. Exemplary heteroatoms include N, O, and S. In most instances, one or two carbon atoms of the structure of the aryl radicals are replaced by heteroatoms. Exemplary heteroaryls include, without limitation, pyridyl, pyrimidyl, pyrazyl, triazyl, and five-membered heteroaromatics, such as pyrrole, furan, thiophene, pyrazole, imidazole, triazole, oxazole, thiazole. Heteroaryls may be substituted at none (unsubstituted), one, more than one, or at all substitutable positions. Suitable substituents are as defined above for the aryl radicals.

An "alkyl radical" or "alkyl group" is understood to mean a radical having 1 to 20 carbon atoms, 1 to 10 carbon atoms, or 1 to 8 carbon atoms. The alkyl radical may be branched or unbranched and the carbon chain may optionally be interrupted by one or more heteroatoms, such as N, O, or S. Heteroatoms may have hydrogen substituents and/or any permissible substituents of organic compounds in order to satisfy the valences of the heteroatoms. The alkyl radical may optionally be substituted by one or more of the substituents mentioned for the aryl radicals above. It is also possible that the alkyl radical contain one or more aryl groups thereon, where suitable aryl groups are described above. Exemplary alkyl radicals include, without limitation, methyl, ethyl, i-propyl, n-propyl, i-butyl, n-butyl, t-butyl, sec-butyl, i-pentyl, n-pentyl, sec-pentyl, neopentyl, n-hexyl, i-hexyl and sec-hexyl.

An "alkenyl radical" or "alkenyl group" is understood to mean a radical having 2 to 20 carbon atoms, 2 to 10 carbon atoms, or 2 to 8 carbon atoms, which may be optionally substituted and at least one carbon-carbon double bond.

An "alkynyl radical" or "alkynyl group" is understood to mean a radical having 2 to 20 carbon atoms, 2 to 10 carbon atoms, or 2 to 8 carbon atoms, which may be optionally substituted and at least one carbon-carbon triple bond.

A "cycloalkyl radical" or "cycloalkyl group" is understood to mean a cyclic radical having 3 to 20 carbon atoms, 3 to 10 carbon atoms, or 3 to 8 carbon atoms. The carbon chain of the cycloalkyl radical may optionally be interrupted by one or more heteroatoms, such as N, O, or S. Heteroatoms may have hydrogen substituents and/or any permissible substituents of organic compounds in order to satisfy the valences of the heteroatoms. The cycloalkyl radical may be unsubstituted or substituted, i.e. substituted by one or more of the substituents mentioned herein.

A "cycloalkenyl radical" or "alkenyl group" is understood to mean a cyclic radical having 4 to 20 carbon atoms, 4 to 10 carbon atoms, or 4 to 8 carbon atoms, which may be optionally substituted and at least one carbon-carbon double bond.

A "cycloalkynyl radical" or "cycloalkynyl group" is understood to mean a cyclic radical having 6 to 20 carbon atoms, 6 to 10 carbon atoms, or 6 to 8 carbon atoms, which may be optionally substituted and at least one carbon-carbon triple bond.

"Carbonyl group," as used herein, is understood to mean moieties which can be represented by the general formula: 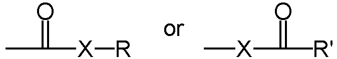 wherein X is a bond, or represents an oxygen or a sulfur, and R represents a hydrogen, a substituted or unsubstituted alkyl, substituted or unsubstituted alkenyl, substituted or unsubstituted alkynyl, substituted or unsubstituted cycloalkyl, substituted or unsubstituted aryl, or substituted or unsubstituted heteroaryl, -(CH₂)ₘ-R''; wherein R' represents a hydrogen, substituted or unsubstituted alkyl, substituted or unsubstituted alkenyl, substituted or unsubstituted alkynyl, substituted or unsubstituted cycloalkyl, substituted or unsubstituted aryl, or substituted or unsubstituted heteroaryl or -(CH₂)ₘ-R''; wherein R'' represents a hydroxy group, substituted or unsubstituted carbonyl group, an aryl, a cycloalkyl, a heterocycle, or a polycycle; and m is zero or an integer ranging from 1 to 8. Where X is oxygen and R is defined as above, the moiety can be referred to as a "carboxyl group." When X is oxygen and R is hydrogen, the formula represents a "carboxylic acid group." Where X is oxygen and R' is hydrogen, the formula represents a "formate group." Where X is oxygen and R or R' is not hydrogen, the formula represents an "ester group." In general, where the oxygen atom of the above formula is replaced by a sulfur atom, the formula represents a "thiocarbonyl group." Where X is sulfur and R or R' is not hydrogen, the formula represents a "thioester group." Where X is sulfur and R is hydrogen, the formula represents a "thiocarboxylic acid group." Where X is sulfur and R' is hydrogen, the formula represents a "thioformate group." Where X is a bond and R is not hydrogen, the above formula represents a "ketone group." Where X is a bond and R is hydrogen, the above formula represents an "aldehyde group." The term "substituted carbonyl" refers to a carbonyl, as defined above, wherein one or more hydrogen atoms in R, R' or a group to which the moiety is attached, are independently substituted with suitable substituents, as defined below.

An "amide group" or "amido" is understood to mean a moiety represented by the general formula: 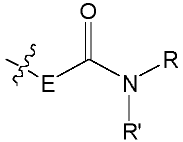 wherein, E is absent, or E is substituted or unsubstituted alkyl, substituted or unsubstituted alkenyl, substituted or unsubstituted alkynyl substituted or unsubstituted cycloalkyl, substituted or unsubstituted aryl, substituted or unsubstituted heteroaryl, wherein independently of E, R and R' each independently represent a hydrogen, substituted or unsubstituted alkyl, substituted or unsubstituted alkenyl, substituted or unsubstituted alkynyl, substituted or unsubstituted carbonyl, substituted or unsubstituted cycloalkyl, substituted or unsubstituted aryl, or substituted or unsubstituted heteroaryl, -(CH₂)ₘ-R‴, or R and R' taken together with the N atom to which they are attached complete a heterocycle having from 3 to 14 atoms in the ring structure; R‴ can represent a hydroxy group, substituted or unsubstituted carbonyl group, an aryl group, a cycloalkyl group, a heterocycle, or a polycycle; and m is zero or an integer ranging from 1 to 8. When E is oxygen, a "carbamate group" is formed. The carbamate cannot be attached to another chemical species, such as to form an oxygen-oxygen bond, or other unstable bonds, as understood by one of ordinary skill in the art.

The term "substituted," as used herein, refers to all permissible substituents of the compounds or functional groups described above. Exemplary substituents include, but are not limited to, halogens, hydroxyl groups, or any other organic groupings containing any number of carbon atoms, preferably 1-14 carbon atoms, and optionally include one or more heteroatoms, such as oxygen, sulfur, or nitrogen grouping in linear, branched, or cyclic structural formats. Representative substituents can include alkyl, substituted alkyl (such as -CF₃ and -CD₃), alkenyl, substituted alkenyl, alkynyl, substituted alkynyl, phenyl, substituted phenyl, aryl, substituted aryl, heteroaryl, substituted heteroaryl, halo, hydroxyl, alkoxy, formyl, substituted alkoxy, phenoxy, substituted phenoxy, aroxy, substituted aroxy, thio (-SH), substituted thio, arylthio, substituted arylthio, cyano, isocyano, substituted isocyano, carbonyl, substituted carbonyl, carboxyl, substituted carboxyl, carboxylates, amino, substituted amino, amide, substituted amide, sulfonyl, substituted sulfonyl, sulfonic acid, phosphoryl, substituted phosphoryl, phosphonyl, substituted phosphonyl, polyaryl, substituted polyaryl, cyclic (such as C₃-C₂₀ cyclic), substituted cyclic (such as substituted C₃-C₂₀ cyclic), heterocyclic, substituted heterocyclic, deuterium, trihaloalkyl (trifluoromethyl), unsubstituted diarylamino, substituted diarylamino, unsubstituted dialkylamino, substituted dialkylamino, azo, carbonate ester, nitro, nitroso, phosphino, pyridyl, NRR', SR, C(O)R, COOR, C(O)NR, SOR, and SOR groups, wherein R and R' are independently selected from hydrogen atom, deuterium atom, or any of the substituents named above.

The numerical ranges disclose individually each possible number that such a range could reasonably encompass, as well as any sub-ranges and combinations of sub-ranges encompassed therein. For example, in a given range concentration range of 10 µM to 500 µM, the range also discloses 20, 33, 350, and 499 µM, as well as any subrange between these numbers (for example, 50 µM to 250 µM), and any possible combination of ranges possible between these values.

Use of the term "about" is intended to describe values either above or below the stated value, which the term "about" modifies, to be within a range of approximately +/-10%. When the term "about" is used before a range of numbers (*i.e*., about 1-5) or before a series of numbers (*i.e*., about 1, 2, 3, 4, etc.) it is intended to modify both ends of the range of numbers and/or each of the numbers recited in the entire series, unless specified otherwise.

### II. Metal-Based Antibody-Drug Conjugates (ADCs) and Pharmaceutical

### Compositions Thereof

Described herein are metal-based antibody-drug conjugates (denoted ADCs), as well as pharmaceutical compositions thereof.

In some non-limiting instances, a metal-based antibody-drug conjugate includes:
a gold(III) porphyrin;
a linker group; and
an antibody;
where the linker group covalently links the metalloporphyrin and the antibody.

The gold(III) porphyrin acts as a payload in the ADCs described. In some instances, the metal-based antibody-drug conjugate has a chemical structure according to Formula (I): 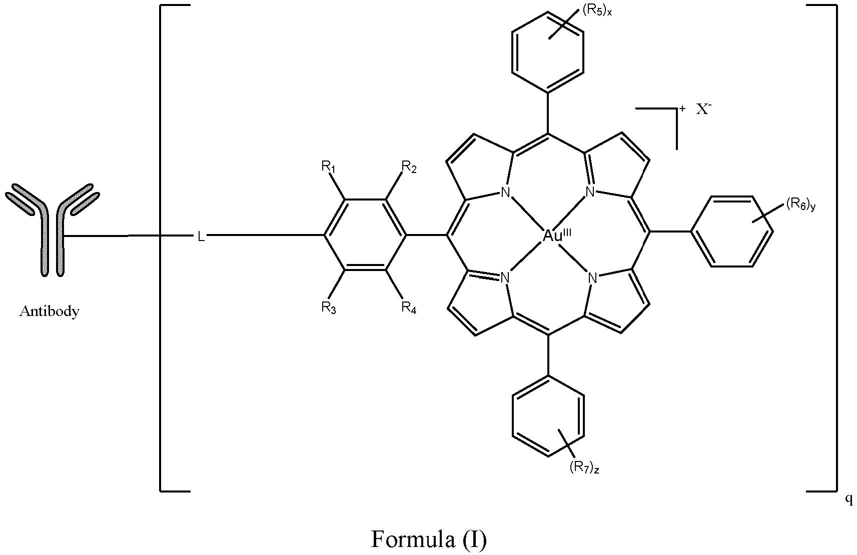
where L is the linker group;
where R₁, R₂, R₃, and R₄ are each independently selected from a hydrogen; halogen group (i.e., -F, -Cl, -Br, -I); a C₂-C₅ alkyl group (linear or branched), such as a methyl, ethyl, propyl, butyl, or pentyl group; alkenyl group; alkynyl group; cycloalkyl group; cycloalkenyl group; cycloalkynyl group; a hydroxyl group; an alkoxy group, such as methoxy, ethoxy, propoxy, or butoxy; an aryl group (i.e., a phenyl group); a heteroaryl group; a benzyl group; an acyl group; an ester group; a carbonyl group; a carboxylate group; an amino group (primary, secondary, or tertiary); an amide group; and a nitro group;
where R₅, R₆, and R₇ are optional substituents and are each independently selected from a halogen group (i.e., -F, -Cl, -Br, -I); a C₂-C₅ alkyl group (linear or branched), such as a methyl, ethyl, propyl, butyl, or pentyl group; alkenyl group; alkynyl group; cycloalkyl group; cycloalkenyl group; cycloalkynyl group; a hydroxyl group; an alkoxy group, such as methoxy, ethoxy, propoxy, or butoxy; an aryl group (i.e., a phenyl group); a heteroaryl group; a benzyl group; an acyl group; an ester group; a carbonyl group; a carboxylate group; an amino group (primary, secondary, or tertiary); an amide group; and a nitro group, ; and x, y, and z are integer values independently selected from 0, 1, 2, 3, 4, and 5;

wherein X⁻ is a pharmaceutically acceptable counterion; and
wherein q is a value in a range of about 1 to 8, or individual values or sub-ranges contained within. In some instances, q is 4 or 8. The value q is understood to represent the number of linker-drug units attached to an antibody where there may be 1, 2, 3, 4, 5, 6, 7, or 8 units attached, on average, to each antibody.

For ADCs of Formula (I), the pharmaceutically acceptable counterion X⁻ can be any suitable pharmaceutically acceptable counterion, such as a chloride ion. It is understood that the ADC of Formula I should be neutral.

In some instances, R₁, R₂, R₃, and R₄ are each hydrogen and/or x, y, and z are each 0. It is further understood that for the ADCs of Formula (I) when x, y, and/or z are 0 it means that no substituents are present and that hydrogens are present on the phenyl rings shown.

In some instances, the metal ion is preferably gold(III). In particular, it is believed that conjugation of tumor-targeting antibody with a gold complex could provide an effective ADC for targeted therapy, such as for cancer. Gold complexes have demonstrated a variety of anticancer properties, such as inhibition of thioredoxin reductase and proteasome, direct DNA damage, alteration of cell cycles and modulation of specific kinases. In particular, gold(III) porphyrin complexes have demonstrated excellent stability under physiological conditions (Che, *et al.* A multi-functional PEGylated gold(iii) compound: potent anti-cancer properties and self-assembly into nanostructures for drug co-delivery. Chem. Sci., 2017, 8, 1942-1953). Therefore, gold(III) porphyrins have received growing attention in the past few decades but, by themselves, have shown poor bioavailability and toxicity issues in normal cells that have limited their clinical development.

In some instances, the linker group includes a cleavable bond, such as an acid cleavable bond, therein; and/or the linker group includes one or more polyethylene glycol (PEG) groups; and/or the linker group includes a spacer groups, such as p-aminobenzyl (PAB) and amino methylene (AM) spacer. The ADC can be internalized into cancer cells by receptor-mediated endocytosis. The acid cleavable bond of the linker group can be cleaved in the acidic environments, which is found inside cancer cells, leading to release and delivery of the gold(III) porphyrin drug directly to the cancer cell. In some instances, the acid cleavable bond is a carbonate bond and the cathepsin B cleavable peptide is GGFG. Other non-limiting examples of cleavable bonds include acid-cleavable hydrazones and acetals; glutathione-cleavable disulfides; and enzyme-cleavable Valine-Citrulline, Valine-Alanine, Phenylalanine-Lysine, and Glycine-Glycine-Phenylalanine-Glycine, which are known in the art. In some instances, non-cleavable linkers are used, for example, SMCC and EMCS. In some instances, the linker group includes a maleimide or N-hydroxysuccinimide moiety which is conjugated with one or more of the thiol or amino group of antibodies, respectively. Non-limiting examples of cleavable and non-cleavable moieties are denoted in the structures below:

### Cleavable Linkers:

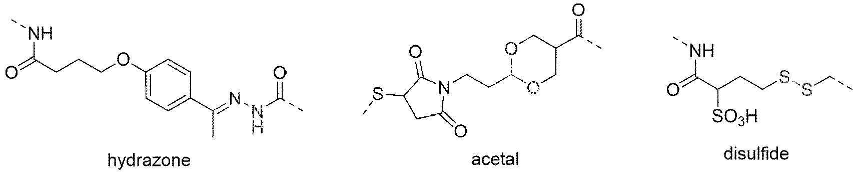

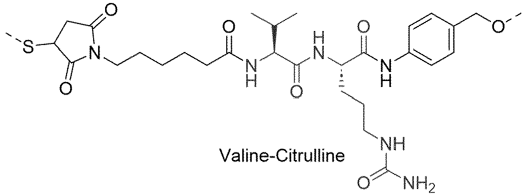

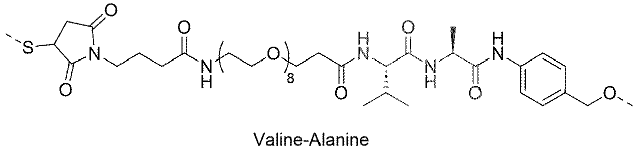

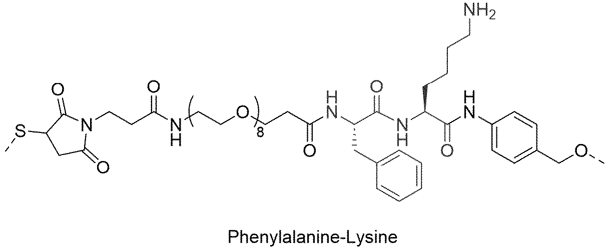

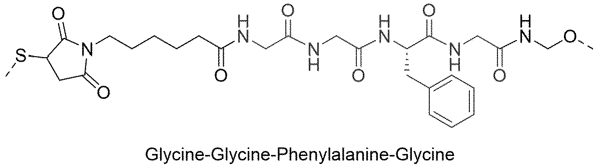

### Non-cleavable Linkers:

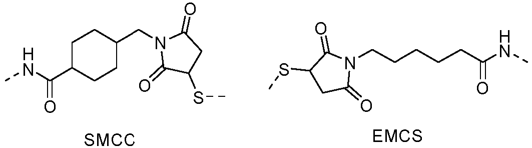

In some instances, the linker group L has a chemical structure according to a structure of Formula (II) shown below: 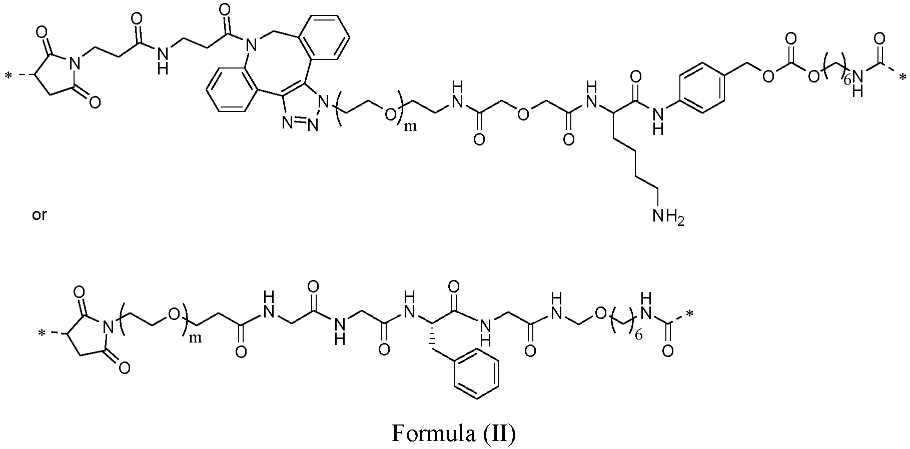 where m is an integer value in a range from 4 to 10, as well as individual values or sub-ranges contained within the aforementioned range. In some instances, m is 8.

In some instances, the antibody is specific for an antigen associated with cells or tissue where the gold(III) porphyrin is to be targeted. In some instances, the antibody is a monoclonal antibody targeting tumor-associated receptors, including TROP2, HER2, Nectin4, folate receptor alpha, and EGFR. In some instances, the monoclonal antibody is a TROP2-targeted Sacituzumab, an EGFR-targeted Cetuximab, and a HER2-targeted Trastuzumab. These receptors are therapeutic targets of interest for use in cancer treatment, as it is overexpressed in a range of malignant tumors and associated with tumor development, invasion, and metastasis. Thus, an ADC having a tumor-targeting monoclonal antibody thereon can be used for binding with the overexpressed tumor-associated receptors on a cancer cell surface and can be internalized through receptor-mediated endocytosis into lysosomes and further digested by the acidic microenvironment or lysosomal proteases, leading to linker cleavage and drug release. Thus, in some particular instances, the metal-based antibody-drug conjugate has the following structures: 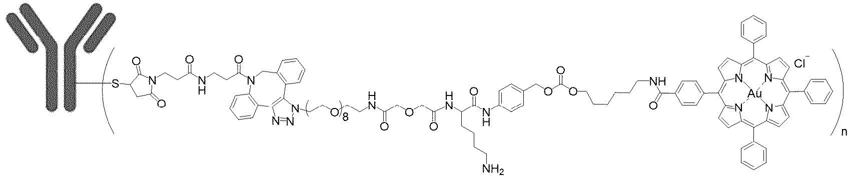 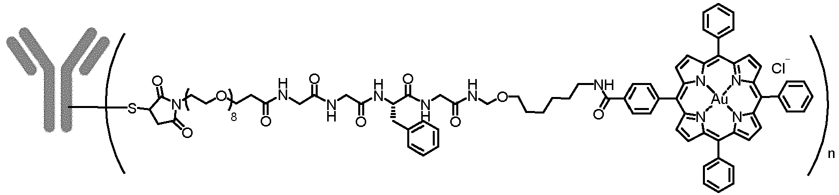 where n is has a value of about 1 to 8 or is about 4 or 8; and where the ADC can be synthesized by conjugating a hydroxy-substituted gold(III) tetraphenylporphyrin (AuTPP) with a tumor-targeting monoclonal antibody, such as TROP2-targeted Sacituzumab, EGFR-targeted Cetuximab and HER2-targeted Trastuzumab, via a linker group including a maleimide moiety, a PEG unit, and an acid-cleavable carbonate linker or a cathepsin B-cleavable tetrapeptide GGFG.

### A. Pharmaceutical Compositions

In some instances, the metal-based antibody-drug conjugates (ADCs) described herein, forms part of a pharmaceutical composition. The pharmaceutical composition includes an effective amount of the ADC and optionally one or more pharmaceutically acceptable carriers and/or excipients.

In one non-limiting instance, a pharmaceutical composition includes:
a plurality of the metal-based antibody-drug conjugates described above;
one or more pharmaceutically acceptable carriers; and
optionally one or more pharmaceutically acceptable excipients.

In some instances, the one or more pharmaceutically acceptable carriers are selected from water, ethanol, polyethylene glycol, propylene glycol, chitosan polymers and chitosan derivatives (e.g. N-trimethylene chloride chitosan, chitosan esters, chitosan modified with hydrophilic groups, such as amino groups, carboxyl groups, sulfate groups, etc.), methylcellulose, an oil, and combinations thereof. Other suitable carriers are known.

The pharmaceutical compositions include an effective amount of the ADC to treat cancer, such as a cancer where the cells overexpress TROP2, EFGR, or HER2 receptor. In some instances, the ADC is present in a concentration ranging from about 1 µM to about 1 mM, from about 10 µM to about 500 µM, from about 10 µM to about 200 µM, or from about 25 µM to about 100 µM of the pharmaceutical composition, as well as individual values and sub-ranges contained within the aforementioned ranges. In some other instances, the ADC is present in a concentration ranging from about 1 µM to about 10 mM, from about 1 µM to about 5 mM, from about 10 µM to about 1 mM, or from about 10 µM to about 500 µM of the pharmaceutical composition, as well as individual values and sub-ranges contained within the aforementioned ranges.

In some instances, depending on the route of administration, an effective amount of the ADC in the composition can be between 0.01 to 1000 mg per kilogram body weight of the subject per day, between 0.1 and 500 mg, such as between 1 and 250 mg, for example about 5, 10, 20, 50, 100, 150, 200 or 250 mg, per kilogram body weight of the subject per day, which can be administered as a single daily dose, divided over one or more daily doses. The amount of the ADC administered, the route of administration, and the further treatment regimen can be determined by the treating clinician or testing technologies, depending on factors such as the age, gender and general condition of the subject, the nature and severity of the disease/symptoms being prevented, treated, or diagnosed, and/or the samples being tested.

Pharmaceutical compositions containing a therapeutically effective amount of the ADC described, and optionally one or more additional therapeutic, prophylactic, and/or diagnostic agents are provided. Pharmaceutical compositions can be for administration by parenteral (intramuscular, intraperitoneal, intravenous (IV), or subcutaneous injection), enteral, or transmucosal (intranasal, vaginal, rectal, or sublingual) routes of administration or using bioerodible inserts and can be formulated in dosage forms appropriate for each route of administration.

### i. Parenteral Compositions

Pharmaceutical compositions containing the ADC described can be administered in an aqueous solution, by parenteral injection. Such compositions may also be in the form of a suspension or emulsion. In general, pharmaceutical compositions are provided including effective amounts of the active agent(s) (i.e., the ADC and optionally include pharmaceutically acceptable diluents, preservatives, solubilizers, emulsifiers, adjuvants and/or carriers. Such compositions include diluents sterile water, buffered saline of various buffer content (e.g., Tris-HCl, acetate, phosphate), pH and ionic strength; and optionally, additives such as detergents and solubilizing agents (e.g., TWEEN^{®} 20, TWEEN^{®} 80 also referred to as polysorbate 20 or 80), antioxidants (e.g., ascorbic acid, sodium metabisulfite), and preservatives (e.g., Thimersol, benzyl alcohol) and bulking substances (e.g., lactose, mannitol). Examples of non-aqueous solvents or vehicles are propylene glycol, polyethylene glycol, vegetable oils, such as olive oil and corn oil, gelatin, and injectable organic esters such as ethyl oleate. The compositions may be lyophilized and redissolved/resuspended immediately before use. The compositions may be sterilized by, for example, filtration through a bacterium retaining filter, by incorporating sterilizing agents into the compositions, by irradiating the compositions, or by heating the compositions.

These particular aqueous solutions are especially suitable for intranasal or intratracheal administration, such as the respiratory tract. These particular aqueous solutions are also suitable for intravenous, intramuscular, subcutaneous, and intraperitoneal administration. In this connection, sterile aqueous media which can be employed will be known to those of skill in the art in light of the present disclosure. For example, one dosage could be dissolved in 1 ml of isotonic NaCl solution and either added to variation in dosage will necessarily occur depending on the condition of the subject being treated. The person responsible for administration will, in any event, determine the appropriate dose for the individual subject.

### ii. Oral Compositions

The pharmaceutical composition containing the ADC described may be provided in a form suitable for oral administration to a subject in need thereof, such as a mammal (i.e., an oral composition). Oral administration may involve swallowing, so that the ADC and optionally additional active agent(s) enter the gastrointestinal tract, or buccal or sublingual administration may be employed by which the ADC enter the blood stream directly from the mouth.

Compositions suitable for oral administration include solid compositions such as tablets, capsules containing particulates, liquids, powders, lozenges (including liquid-filled lozenges), chews, multi- and nano-particulates, gels, solid solutions, liposomes, films, ovules, sprays, and liquid compositions.

Liquid compositions for oral administration include suspensions, solutions, syrups, and elixirs. Such oral compositions may be employed as fillers in soft or hard capsules and can contain one or more suitable carriers and/or excipients, for example, water, ethanol, polyethylene glycol, propylene glycol, chitosan polymers and chitosan derivatives (e.g. N-trimethylene chloride chitosan, chitosan esters, chitosan modified with hydrophilic groups, such as amino groups, carboxyl groups, sulfate groups, etc.), methylcellulose, a suitable oil, one or more emulsifying agents, and/or suspending agents. Liquid compositions for oral administration may also be prepared by the reconstitution of a solid, for example, from a sachet.

For tablet or capsule dosage forms, in addition to the ADC described herein, tablets generally contain disintegrants, binders, diluents, surface active agents, lubricants, glidants, antioxidants, colourants, flavoring agents, preservatives, or taste masking agents, or a combination thereof.

Examples of suitable disintegrants for forming a table or capsule dosage form containing the ADC can include, but are not limited to, sodium starch glycolate, sodium carboxymethyl cellulose, calcium carboxymethyl cellulose, croscarmellose sodium, crospovidone, polyvinylpyrrolidone, methyl cellulose, microcrystalline cellulose, lower alkyl-substituted hydroxypropyl cellulose, starch, pregelatinised starch and sodium alginate. Generally, the disintegrant can have a concentration in a range from about 1 wt% to about 25 wt%, from about 5 wt% to about 20 wt% of the tablet or capsule dosage form containing the ADC.

Binders are generally used to impart cohesive qualities to a tablet composition containing the ADC. Suitable binders for forming a tablet or capsule formulation can include, but are not limited to, microcrystalline cellulose, gelatin, sugars, polyethylene glycol, natural and synthetic gums, polyvinylpyrrolidone, pregelatinised starch, chitosan polymers and chitosan derivatives (e.g. N-trimethylene chloride chitosan, chitosan esters, chitosan modified with hydrophilic groups, such as amino groups, carboxyl groups, sulfate groups, etc.), hydroxypropyl cellulose, and hydroxypropyl methylcellulose.

Suitable diluents for forming a table or capsule composition containing the ADC can include, but are not limited to, lactose (as, for example, the monohydrate, spray-dried monohydrate or anhydrous form), chitosan polymers and chitosan derivatives (e.g. N-trimethylene chloride chitosan, chitosan esters, chitosan modified with hydrophilic groups, such as amino groups, carboxyl groups, sulfate groups, etc.), N-sulfonated derivatives of chitosan, quaternarized derivatives of chitosan, carbosyalkylated chitosan, microcrystalline chitosan, mannitol, xylitol, dextrose, sucrose, sorbitol, microcrystalline cellulose, starch and dibasic calcium phosphate dihydrate.

Tablet or capsule compositions containing the ADC may also contain surface active agents, such as sodium lauryl sulfate and polysorbate 80, and glidants such as silicon dioxide and talc, in the coating. When present, surface active agents can have a concentration in a range from about 0.2 wt% to 5 wt% of the tablet or capsule formulation.

Tablet or capsule compositions containing the ADC also generally contain lubricants, such as magnesium stearate, calcium stearate, zinc stearate, sodium stearyl fumarate, and mixtures of magnesium stearate with sodium lauryl sulphate. Lubricants can have a concentration in a range from about 0.25 wt% to 10 wt%, from about 0.5 wt% to about 3 wt% of the tablet or capsule composition.

Other possible excipients included in a tablet or capsule formulation containing the ADC include glidants (e.g. Talc or colloidal anhydrous silica at about 0.1 wt% to about 3 wt% of the table or capsule formulation), antioxidants, colourants, flavouring agents, preservatives, and taste-masking agents. When present, glidants can have a concentration in a range from about 0.2 wt% to 1 wt% of the tablet or capsule composition.

Tablet or capsule blends, including the ADCand one or more suitable excipients, may be compressed directly or by roller to form tablets. Tablet or capsule blends or portions of the blends may alternatively be wet-, dry-, or melt-granulated, melt congealed, or extruded before tableting. The final table or capsule composition may contain one or more layers and may be coated or uncoated; it may even be encapsulated in a particle, such as a polymeric particle or a liposomal particle.

Solid formulations containing the ADC for oral administration may be formulated to be immediate and/or modified release. Modified release formulations include delayed, sustained, pulsed, controlled, targeted and programmed release formulations.

### iii. Optional Therapeutic, Prophylactic or Diagnostic Agents

In some forms, the pharmaceutical compositions can optionally include one or more additional active agents. Therefore, in some forms, the pharmaceutical composition includes the ADC, in addition to at least one of a therapeutic, prophylactic, and/or diagnostic agents. The additional agents can be included together with the ADC or may be a separate composition for co-administration.

Non-limiting examples of therapeutic, prophylactic, or diagnostic agents can include bronchodilators, corticosteroids, methylxanthines, phosphodiesterase-4 inhibitors, anti-angiogenesis agents, antibiotics, antioxidants, anti-viral agents, anti-fungal agents, anti-inflammatory agents, immunosuppressant agents, anti-allergic agents, and combinations thereof. The amount of any additional therapeutic, prophylactic, or diagnostic agents generally depends on the severity of the diseases and/or disorders to be treated. Specific dosages can be readily determined by those of skill in the art. See Ansel, Howard C. et al., Pharmaceutical Dosage Forms and Drug Delivery Systems (6th ed.) Williams and Wilkins, Malvern, PA (1995).

### iv. Carriers and Excipients

The pharmaceutical compositions may be formulated with one or more excipients and/or carriers appropriate to the indicated route of administration. In some forms, the ADC is formulated in a manner amenable for the treatment of human and/or veterinary subjects. In some forms, the pharmaceutical compositions include admixing or combining the ADC with one or more of the following excipients: lactose, sucrose, starch powder, cellulose esters of alkanoic acids, cellulose alkyl esters, talc, stearic acid, magnesium stearate, magnesium oxide, sodium and calcium salts of phosphoric and sulfuric acids, gelatin, acacia, sodium alginate, polyvinyl-pyrrolidone, and/or polyvinyl alcohol. In some forms, e.g., for oral administration, the pharmaceutical compositions may be tableted or encapsulated. In some forms, the ADC may be slurried in water, polyethylene glycol, propylene glycol, ethanol, corn oil, cottonseed oil, peanut oil, sesame oil, benzyl alcohol, sodium chloride, and/or various buffers. In some forms, the pharmaceutical compositions may be subjected to pharmaceutical operations, such as sterilization, and/or may contain carriers and/or excipients such as preservatives, stabilizers, wetting agents, emulsifiers, encapsulating agents such as lipids, dendrimers, polymers, proteins such as albumin, nucleic acids, and buffers.

### v. Dosage Forms

In some forms, it may be advantageous to formulate pharmaceutical compositions in dosage unit form for ease of administration and uniformity of dosage. "Dosage unit form" as used herein refers to physically discrete units suited as unitary dosages for the patients to be treated; each unit containing a predetermined quantity of therapeutic agent (i.e., the ADC) calculated to produce the desired therapeutic effect in association with a pharmaceutical carrier. In some forms, the specification for the dosage unit forms dictated by and directly dependent on (a) the unique characteristics of the therapeutic agent and the particular therapeutic effect to be achieved, and (b) the limitations inherent in the art of compounding such a therapeutic agent for the treatment of a selected condition in a patient. In some forms, the ADC are administered at a therapeutically effective dosage sufficient to treat a condition, such as cancer, associated with a condition in a patient. For example, the efficacy of a pharmaceutical composition containing the ADC can be evaluated in an animal model system that may be predictive of efficacy in treating the disease in a human or another animal.

In some forms, the effective dose range for the therapeutic agent can be extrapolated from effective doses determined in animal studies for a variety of different animals. Precise amounts of the pharmaceutical composition depend on the judgment of the practitioner and are specific to each individual. Other factors affecting the dose include the physical and clinical state of the subject, the route of administration, the intended goal of treatment and the potency, stability, and toxicity of the particular pharmaceutical composition.

The actual dosage amount of an antibiotic composition of the present disclosure administered to a subject may be determined by physical and physiological factors such as type of animal treated, age, sex, body weight, severity of condition, the type of condition being treated, previous or concurrent therapeutic interventions, idiopathy of the patient and on the route of administration. These factors may be determined by a skilled artisan. The practitioner responsible for administration will typically determine the concentration of active agent(s) in a composition and appropriate dose(s) for the individual subject. The dosage may be adjusted by the individual physician in the event of any complication.

Single or multiple doses of the active agent(s), such as the ADC, are contemplated. Desired time intervals for delivery of multiple doses can be determined by one of ordinary skill in the art employing no more than routine experimentation. As an example, patients may be administered two doses daily at approximately 12-hour intervals. In some forms, the agent is administered once a day.

The pharmaceutical composition may be administered on a routine schedule. As used herein, a routine schedule refers to a predetermined designated period of time. The routine schedule may encompass periods of time which are identical, or which differ in length, as long as the schedule is predetermined. For instance, the routine schedule may involve administration twice a day, every day, every two days, every three days, every four days, every five days, every six days, a weekly basis, a monthly basis or any set number of days or weeks there-between. Alternatively, the predetermined routine schedule may involve administration on a twice daily basis for the first week, followed by a daily basis for several months, etc. In other forms, the invention provides that the agent(s) may be taken orally and that the timing of which is or is not dependent upon food intake. Thus, for example, the agent can be taken every morning and/or every evening, regardless of when the patient has eaten or will eat.

In some forms, the pharmaceutical composition can be in a unit dosage form which can be suitably packaged, for example in a box, blister, vial, bottle, sachet, ampoule or in any other suitable single-dose or multi-dose holder or container (which can be properly labeled); optionally with one or more leaflets containing product information and/or instructions for use. Generally, such unit dosages can contain between 1 and 1000 mg, or between 5 and 500 mg, of the disclosed ADC, e.g., about 10, 25, 50, 100, 200, 300 or 400 mg per unit dosage.

### III. Methods of Making Metal-Based Antibody-Drug Conjugates (ADCs)

The metal-based antibody-drug conjugates described herein can be synthesized according to the methods described below. In one non-limiting instances, a method of making a metal-based antibody-drug conjugate includes the steps of:
(a) forming or providing a gold(III) porphyrin including a linker group; wherein the linker group comprises an end-group known as an antibody-attachment moiety, such as a thiol-reactive Michael acceptor group and amine-reactive N-Hydroxysuccinimide group; and/or the linker group comprises a spacer, and/or the linker group comprises a cleavable bond, such as an acid cleavable bond and cathepsin B cleavable peptide therein; and
(b) conjugating the gold(III) porphyrin to an antibody, such as a monoclonal antibody, to form the metal-based antibody-drug conjugate;
   wherein the monoclonal antibody comprises one or more thiol groups and at least one of the thiol groups undergoes a Michael addition with the Michael acceptor group of the linker group; or the monoclonal antibody includes one or more amino groups and at least one of the amino groups undergoes a condensation reaction with the N-hydroxysuccinimide of the linker group.

In some instances of the methods, the gold(III) porphyrin has a structure according to Formula (III): 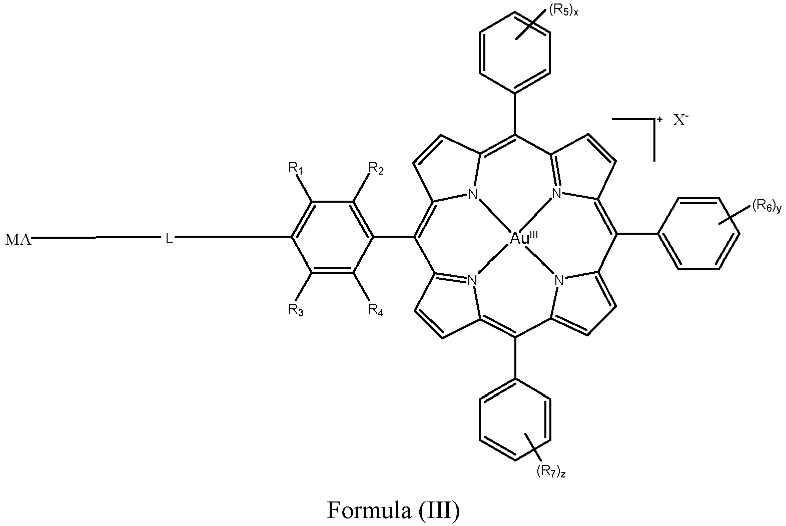
where L is a linker group;
where MA is a Michael acceptor group;
where R₁, R₂, R₃, and R₄ are each independently selected from the group consisting of a hydrogen; halogen group (i.e., -F, -Cl, -Br, -I); a C₂-C₅ alkyl group (linear or branched), such as a methyl, ethyl, propyl, butyl, or pentyl group; alkenyl group; alkynyl group; cycloalkyl group; cycloalkenyl group; cycloalkynyl group; a hydroxyl group; an alkoxy group, such as methoxy, ethoxy, propoxy, or butoxy; an aryl group (i.e., a phenyl group); a heteroaryl group; a benzyl group; an acyl group; an ester group; a carbonyl group; a carboxylate group; an amino group (primary, secondary, or tertiary); an amide group; and a nitro group;
where R₅, R₆, and R₇ are optional substituents and are each independently selected from the group consisting of a halogen group (i.e., -F, -Cl, -Br, -I); a C₂-C₅ alkyl group (linear or branched), such as a methyl, ethyl, propyl, butyl, or pentyl group; alkenyl group; alkynyl group; cycloalkyl group; cycloalkenyl group; cycloalkynyl group; a hydroxyl group; an alkoxy group, such as methoxy, ethoxy, propoxy, or butoxy; an aryl group (i.e., a phenyl group); a heteroaryl group; a benzyl group; an acyl group; an ester group; a carbonyl group; a carboxylate group; an amino group (primary, secondary, or tertiary); an amide group; and a nitro group, ; and x, y, and z are integer values independently selected from 0-5;
   and
wherein X⁻ is independently a pharmaceutically acceptable counterion.

In some instances, the Michael acceptor group can be replaced with another sulfhydrl (thiol) reactive group.

In some instances, R₁, R₂, R₃, and R₄ are each hydrogen and/or x, y, and z are each 0.

In some instances, the linker group comprises a cleavable bond, such as an acid cleavable bond and/or an enzyme-cleavable linker, therein; and/or the linker group comprises one or more polyethylene (PEG) groups. In certain instances, the acid cleavable bond is a carbonate bond and the enzyme-cleavable linker is a cathepsin B cleavable peptide, such as Glycine-Glycine-Phenylalanine-Glycine (GGFG).

In some instances, functionalizing the gold(III) porphyrin for bioconjugation involves reacting a gold(III) porphyrin having a carboxylic acid group thereon with coupling it to an amine-containing molecule having a hydroxy group thereon, such as 6-amino-1-hexanol, to produce a gold(III) porphyrin having a hydroxyl group. The skilled person is familiar with methods for coupling amine-containing molecules with carboxylic acids, as well as alternative coupling methods to afford a gold(III) porphyrin having a hydroxyl group thereon. The skilled person is also familiar with methods for synthesizing any hydroxyl-substituted gold(III) porphyrin. Next, the gold(III) porphyrin having a hydroxyl group can be coupled to a molecule containing a p-aminobenzyl alcohol and a terminal azide group, such as N₃-PEG-Lysine(Mmt)-PABOH, where the molecule reacts with the hydroxyl group, such as in the presence of triphosgene, to afford a cleavable bond, such as a carbonate bond. Lastly, the azide group is reacted with a molecule containing a strained alkyne and a Michael acceptor group. In some instances, the molecule containing the strained alkyne and the Michael acceptor group is DBCO-maleimide. Thus, in some instances, the linker group is formed using a copper-free strained-promoted azide-alkyne click reaction (SPAAC) *(see* Examples). In some instances, the Michael acceptor group is a maleimide group. In some instances, the non-limiting strained alkynes are DBCO (dibenzocyclooctyne) and BCN (bicyclo[6.1.0]nonyne). In yet another example, the gold(III) porphyrin having a hydroxyl group can be substituted with an acetyl group-appended Fmoc-protected amino acid in the presence of pyridinium p-toluenesulfonate (PPTS). Next, after Fmoc deprotection, it can be coupled to a cathepsin B-cleavable peptide sequence, such as Glycine-Glycine-Phenylalanine-Glycine. Lastly, it is further coupled with 6-maleimidohexanoic acid containing a Michael acceptor group.

In some instances, the linker L has a chemical structure according to one of the following structures of Formula (IV): 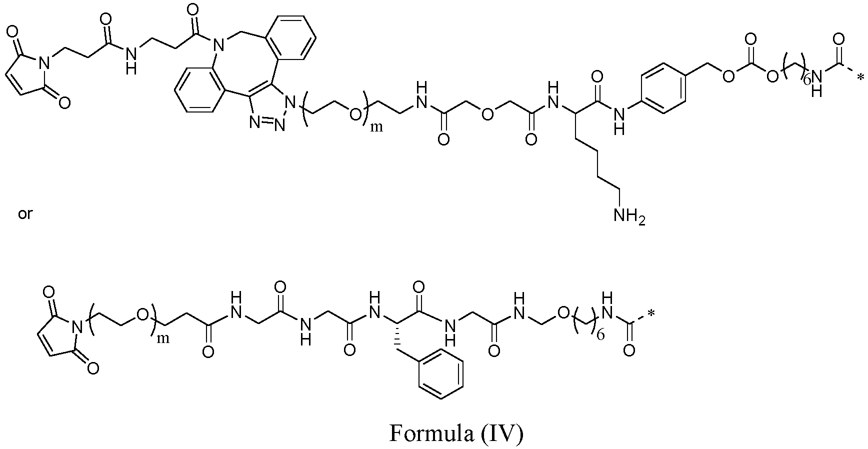 wherein m is an integer value in a range from 4 to 10 and the maleimide group is the Michael acceptor group. In some instances, m is 8.

In some instances of the method, the monoclonal antibody targets tumor-associated receptors, such as TROP2, HER2, Nectin4, folate receptor alpha, and EGFR. In some instances, the monoclonal antibody is a TROP2-targeted monoclonal antibody such as Sacituzumab. In some other instances, the monoclonal antibody is an EGFR-targeted monoclonal antibody, such as Cetuximab. In still other instances, the monoclonal antibody is a HER2-targeted monoclonal antibody, such as Trastuzumab.

In some instances, prior to step (b) of the method the monoclonal antibody is subjected to a reducing treatment which reduces interchain disulfide bonds to afford the one or more thiol groups. Suitable reducing treatments are known. In one instance, the reducing treatment includes exposing the antibody to a reducing agent, such as tris(2-carboxyethyl) phosphine hydrochloride to produce free thiols on the antibody by reduction of interchain disulfide bonds present in the antibody.

For the methods described above, the synthetic conditions (choice of solvent(s), temperatures (such as to achieve refluxing), atmospheres, work-up conditions, purification conditions, etc.) can be varied or modified from those discussed and exemplified herein, as appropriate, by the person of ordinary skill in the art of synthetic chemistry, without impacting the synthesis. The methods may include additional steps, which are not otherwise specified, that may be involved in the syntheses which include, but are not limited to, precipitation of a product, washing the reaction mixture, extracting the reaction mixture, separation of the product from a mixture (e.g. by filtration), drying the product, and combinations thereof. Further processing steps that may be involved include stirring, heating and/or cooling the reaction mixture. Intermediate, crude, and purified products can be characterized using any suitable characterization method known to the skilled person. In some instances, such methods include, but are not limited to, ¹H and ¹³C NMR, mass spectrometry, elemental analysis, or other techniques.

### IV. Methods of Using Metal-Based Antibody-Drug Conjugates (ADCs) and Pharmaceutical Compositions Thereof

The disclosed ADCs can be used to treat cancers, such as cancers where the cells overexpress tumor-associated receptors, such as TROP2, HER2, Nectin4, folate receptor alpha, and EGFR receptors. The ADC is typically administered in a pharmaceutical composition to a subject in need thereof. In one non-limiting example, a method of targeted cancer therapy includes the steps of:
(i) administering a pharmaceutical composition containing a plurality of the ADCs described herein to a subject in need thereof.

In some instances, the subject has a cancer wherein the cancer cells overexpress tumor-associated receptors, such as TROP2, HER2, Nectin4, folate receptor alpha, and EGFR receptors. In some instances, the cancer is selected from breast, lung, liver, kidney, colorectal (such as colon), gastric, ovarian, and pancreatic cancer.

In some instances, the plurality of the metal-based antibody-drug conjugate in the pharmaceutical composition provide a cytotoxicity against the overexpressed tumor-associated receptor, such as in TROP2-overexpressed, EGFR-overexpressed, and HER2-overexpressed cancer cells demonstrating an IC₅₀ value ranging from about 2 to 170 nM, as well as individual values or sub-ranges contained within the aforementioned range.

In certain instances, in step (i) the dosage of the ADC in the pharmaceutical composition administered to the subject is from about 0.1 µg to about 100 µg, from about 0.5 µg to about 50 µg, from about 1 µg to about 100 µg, from about 1 µg to about 50 µg, from about 1 µg to about 25 µg, from about 1 µg to about 10 µg, from about 1 µg to about 5 µg, from about 4 µg to about 10 µg, or from about 1 µg to about 4 µg per gram of the subject's weight, or sub-ranges or individual values contained within these ranges.

The administration in step (i) can be performed using any suitable technique, such as oral administration, intranasal, intramuscular administration, intravenous administration, intraperitoneal administration, or subcutaneous administration, or a combination thereof.

Step (i), administration of pharmaceutical composition, may occur one or more times. When more than one administration step is performed, each administration can be performed regularly every 5 mins, every 10 mins, every 20 mins, every 30 mins, every hour, every 2 hours, every day, every two days, every 3 days, every week, every two weeks, every month, etc.; or irregularly with a time interval of 5 mins, 10 mins, 20 mins, 30 mins, 1 hour, 2 hours, 1 day, 2 days, 3 days, 5 days, 1 week, 2 weeks, etc. The specific number of treatment and time interval for the treatment can be determined by the treating clinician or testing technologies, depending on factors such as the age, gender and general condition of the subject, the nature and severity of the disease/symptoms being prevented, treated, or diagnosed, and/or the samples being tested.

The administration step or all of the administration steps (if repeated), can each provide an effective amount of the ADC in the pharmaceutical composition which is administered to the subject to treat the cancer, such as where the cells overexpress TROP2, EGFR, or HER2 receptors. Optionally, the treatment effect occurs without any notable side effects to the subject.

In some instances of the method, the pharmaceutical composition can further include one or more therapeutic, prophylactic, or diagnostic agents. In some other instances of the method, the method includes co-administering one or more therapeutic, prophylactic, or diagnostic agents to the subject, either before, during, or following step (i).

The present invention will be further understood by reference to the following non-limiting examples.

### EXAMPLES

### Example 1: Sacituzumab-AuOH

### Synthetic Methods and Characterization:

### Synthesis of AuOH:

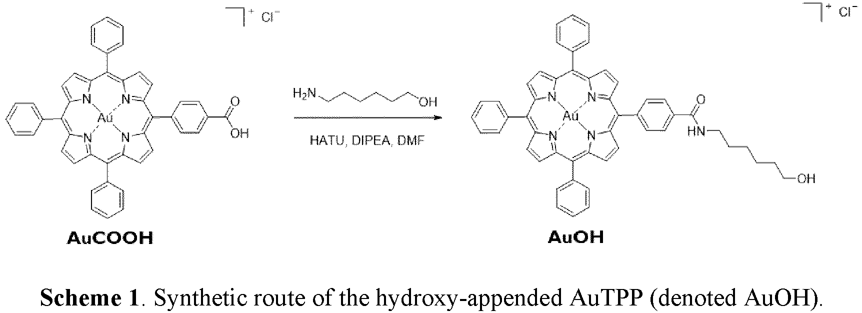

AuCOOH (10 mg, 11.2 µmol) was reacted with 6-amino-1-hexanol (2.6 mg, 22.4 µmol) in the presence of 1-[Bis(dimethylamino)methylene]-1H-1,2,3-triazolo[4,5-b]pyridinium 3-oxid hexafluorophosphate (HATU) (8.6 mg, 22.4 µmol) and N,N-diisopropylethylamine (DIPEA) (8 µL, 44.8 µmol) in dimethylformamide (DMF) (1 mL). The reaction mixture was stirred at room temperature overnight and the solvent was removed in vacuo. The residue was purified by column chromatography on silica gel with CH₂Cl₂/CH₃OH (50:1 v/v) as the eluent to afford AuOH (8.8 mg, 79%).¹H NMR (600 MHz, CDCl₃): δ 9.29-9.30 (m, 8H), 8.24-8.28 (m, 4H), 8.20-8.23 (m, 6H), 7.89-7.93 (m, 3H), 7.85-7.88 (m, 6H), 6.97 (s, 1H), 3.68 (t, *J =* 6 Hz, 2H), 3.56 (q, *J =* 7.2 Hz, 2H), 1.75-1.78 (m, 2H), 1.63-1.65 (m, 2H), 1.50 (br s, 4H). HRMS (ESI): m/z for C₅₁H₄₁AuN₅O₂⁺ [M]⁺ calcd: 952.2926; found 952.2915.

### Synthesis of Linker-AuOH:

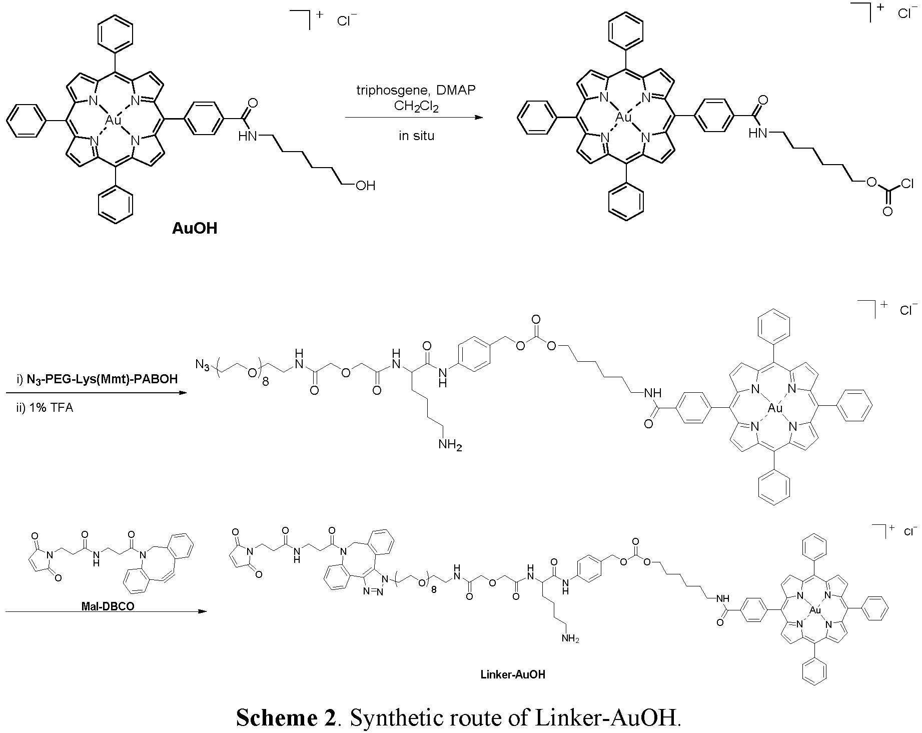

A mixture of AuOH (5 mg, 5.1 µmol), triphosgene (0.75 mg, 2.5 µmol) and 4-dimethylaminopyridine (DMAP) (1.2 mg, 10.2 µmol) in CH₂Cl₂ (5 mL) was stirred at room temperature for 4 h. After the consumption of AuOH, N₃-PEG-Lys(Mmt)-PABOH (5.4 mg, 5.1 µmol) was added to the reaction mixture and stirred overnight, followed by treatment with 1% TFA for 1 h. The solvent was then evaporated under reduced pressure, and the residue was purified by size-exclusion chromatography on Bio-Beads S-X1 beads using CH₂Cl₂ as the eluent. The obtained intermediate was then treated with Mal-DBCO (2.1 mg, 5.1 µmol) in CH₂Cl₂ (2 mL) at room temperature for 1 h. The solvent was then evaporated under reduced pressure, and the residue was purified by size-exclusion chromatography on Bio-Beads S-X1 beads using CH₂Cl₂ as the eluent to afford Linker-AuOH. HRMS (ESI): m/z for C₁₁₂H₁₂₁AuN₁₅O₂₀⁺ [M]⁺ calcd: 2192.8578; found 2192.8636.

### Preparation and Characterization of Sacituzumab-AuOH:

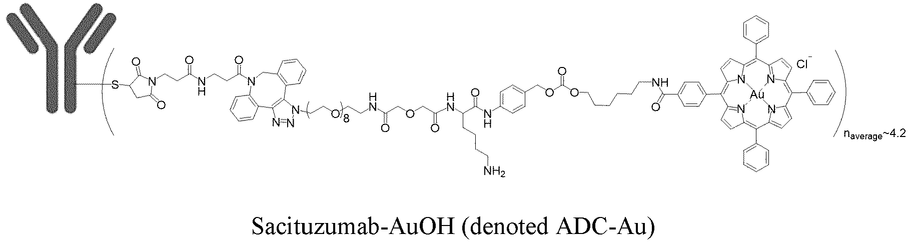

Sacituzumab (MCE, 2.5 mg/mL) was dissolved in borate buffer (pH 8.0) and TCEP (10 equiv.) was added to the reaction mixture for 1.5 h. After the reduction of disulphide bond, Linker-AuOH (12 equiv.) dissolved in DMSO (8% v/v) was added and the reaction mixture was stirred for 2 h. The ADC-Au conjugate was purified by using Zeba^{™} Spin Desalting Column (Thermo Fisher, 7K MWCO) and washed with Amicon Ultra Centrifugal Filter (10 kDa MWCO) according to the manufacturer's protocol. The protein concentration was determined by the standard bicinchoninic acid (BCA) assay and the yield was determined to be 83%. The conjugate was analyzed by Thermo Scientific µDrop and reducing SDS-PAGE with 12% acrylamide with 4% stacking gel as standard.

Sacituzumab and ADC-Au were analyzed on a MAbPac Reversed Phase HPLC Column (4 µm, 3 mm × 50 mm) at a flow rate of 0.3 mL/min. Electrospray ionization (ESI) mass spectra were recorded on an Agilent 6546 LC/Q-TOF mass spectrometer. The condition used for the analysis was set as follows: solvent A = 0.1% FA in deionized water and solvent B = 0.1% FA in acetonitrile; gradient: 80% A + 20% B in the first 0.5 min, then changed to 40% A + 60% B in 3 min, maintained under this condition for 5 min, further changed to 80% A + 20% B in 0.5 min, maintained under this condition for 1 min.
The average drug-to-antibody (DAR) ratio was determined by using the corresponding peak areas of the chromatograms in the deconvoluted mass spectra.

### In Vitro Anti-proliferative Activity (NBB Assay):

Cells (4 × 10³ cells/well) were seeded in a microtiter plate (96 wells) and incubated overnight before the treatment. Different concentrations of AuTPP, AuCOOH, AuOH, ADC-Au and the vehicle control (0.5% DMSO) were added into the wells and then incubated for 72 h. After the media was removed, the cells were fixed with formaldehyde (3%; 50 µL) in PBS and stained with NBB reagent (0.05%, 0.1 M sodium acetate, 9% acetic acid; 50 µL) for overnight. The stained cells in each well were washed thrice gently with deionized water and solubilized in sodium hydroxide solution (50 mM; 100 µL). The cell viability was determined by measuring the absorbance of 620 nm in each well by microplate reader. The experiments were repeated in triplicate.

### Flow Cytometric Study

Cells (2 × 10⁵ cells/tube) were suspended in FACS staining buffer (2% FBS in PBS) into a 1.5 mL eppendorf tube. They were incubated with Sacituzumab, ADC-Au or IgG control respectively in staining buffer (100 µL) at 4 °C. After 1 h, the cell suspensions were centrifuged at 1500 rpm for 3 min at 4 °C and washed with staining buffer (1 mL) twice to discard unbound antibodies. The cells were then incubated with Alexa Fluor 594 goat antihuman secondary antibody at 4 °C for 1 h and then subject to centrifugation. The cells were washed twice again and were resuspended in staining buffer. The mean fluorescence intensities (MFI) were measured using Beckman Cytoflex S Flow Cytometer.

### Confocal Microscopy Study

Cells (1 × 10⁵ cells/tube) were seeded into 35 mm confocal culture. After 24 h of incubation, the media were removed and washed with ice-cold PBS and then placed on ice. The cells were incubated with ADC-Au, which was pre-labelled with Alexa Fluor 647 NHS Ester (Invitrogen) according to the manufacturer's protocol, at 4 °C for 1 h. The solutions were then removed, and the cells were rinsed with ice-cold PBS twice and stained with Hoechst 33342 before being examined with a Zeiss LSM900 Confocal Microscope. To examine the internalization of ADC-Au, the cells were further incubated at 37 °C for 4 h or 24 h. For the subcellular localization, the cells, after being treated with ADC-Au for 24 h, were stained with LysoTracker Green DND-26 to detect lysosomes. For the co-culture experiment, the cells were firstly stained with CellTrace Violet and CellTracker Green CMFDA according to the manufacturer's protocol, before being seeded into confocal dishes.

### In vivo Study

All animal experiments are conducted under the guidelines approved by the Committee on the Use of Live Animals in Teaching and Research of the University of Hong Kong. Female BALB/cAnN-nu (nude) mice (5-8 weeks old) are housed in the Laboratory Animal Unit, The University of Hong Kong. The mice are freely access to food and water. HCC827 lung cancer cells (5 × 10⁶) in PBS (100 µL) are injected into the right flanks of each mouse subcutaneously. Once the tumor volumes reached about 50 mm³, the mice are divided into 5 groups (n = 5 for each group): (A) PBS vehicle control, (B) AuOH (0.3 mg/kg), (C) Sacituzumab (10 mg/kg), (D) ADC-Au (5 mg/kg) and (E) ADC-Au (10 mg/kg). The mice are treated with intravenous administration of a total of four doses on day 0, 5, 9 and 14. The tumor size and body weight are monitored during the treatment. After 21 days, the mice are sacrificed.

### Results and Discussion:

To modify metal complexes, such as a gold(III) porphyri, for bioconjugation, a carboxy-substituted AuTPP (denoted AuCOOH) was condensed with 6-amino-1-hexanol to give the hydroxy-appended AuTPP (denoted AuOH) in the presence of HATU and DIPEA in DMF *(see* Scheme 1 above). The IC₅₀ values of these metal complexes were then examined against a panel of cell lines and compared with the original AuTPP (Table 1 below). As expected, AuCOOH exhibited the lowest cytotoxicity which was attributed to the reduced cellular uptake of the carboxylate group. Interestingly, the potency of AuOH was comparable with the unmodified AuTPP with the submicromolar IC₅₀ values ranging from 0.08 to 0.39 µM, meaning that the modification did not significantly affect the anticancer properties of AuTPP. Therefore, AuOH with the appended hydroxy group was used as a cytotoxic payload to further conjugate with an antibody.

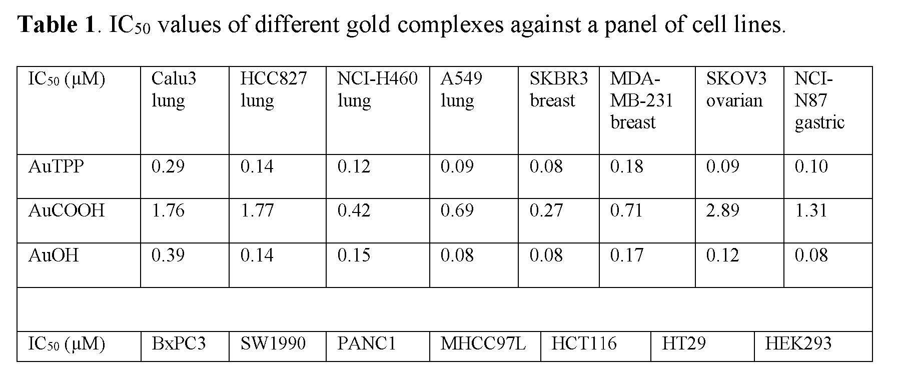

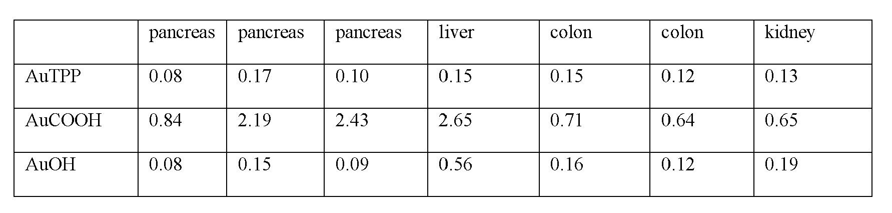

With reference to the design of the FDA-approved Sacituzumab Govitecan, AuOH was conjugated with Sacituzumab via an acid-cleavable carbonate linker. As shown in Scheme 2 above for the synthetic route for the preparation of maleimide-gold porphyrin precursor. AuOH was first treated with triphosgene and 4-(dimethylamino)pyridine (DMAP) to give the corresponding chloroformate *in situ,* which was further condensed with N₃-PEG-Lys(Mmt)-PABOH to form the acid-sensitive carbonate bond. The PEG unit present in the linker can improve the hydrophilicity and solubility of the metal complexes and reduce protein aggregation. After the removal of Mmt protecting group by mild acidic treatment with 1% trifluoroacetic acid (TFA), the azido group was then conjugated with Mal-DBCO through the copper-free SPAAC to afford the final linker-payload conjugate (Linker-AuOH) in quantitative yield.

After reduction of the interchain disulfide bonds of Sacituzumab with tris(2-carboxyethyl) phosphine hydrochloride (TCEP·HCl), Linker-AuOH was conjugated with the free thiol groups via the conventional Michael addition in a borate buffered saline (pH 8.0). After removing the excess Linker-AuOH by Zeba spin desalting column (7 kDa MWCO) and washing with Amicon Ultra Centrifugal Filter (10 kDa MWCO), the resulting ADC Sacituzumab-AuOH (ADC-Au) was obtained with 83% yield as determined by bicinchoninic acid (BCA) protein assay.

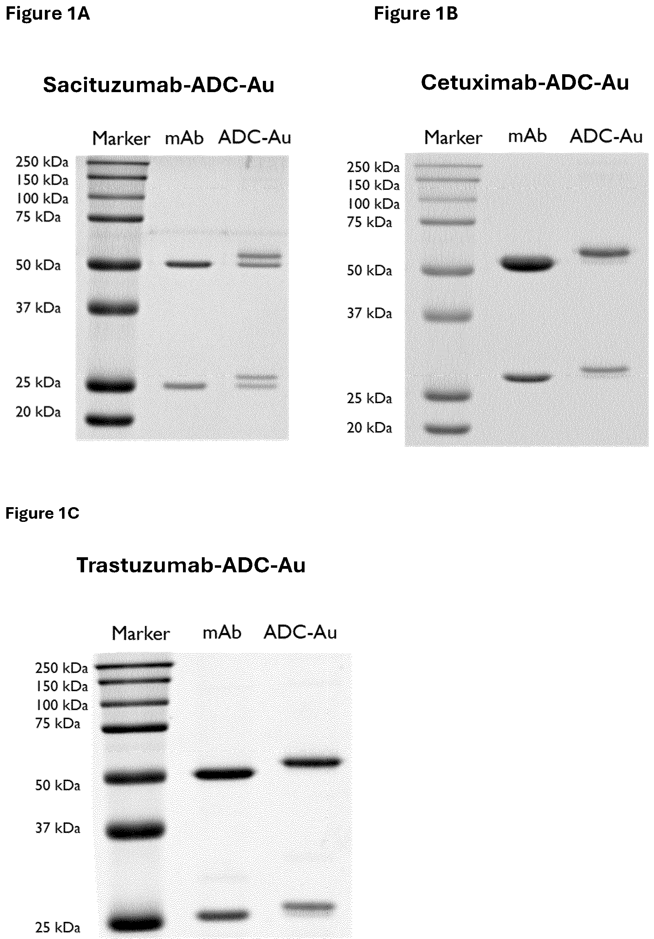

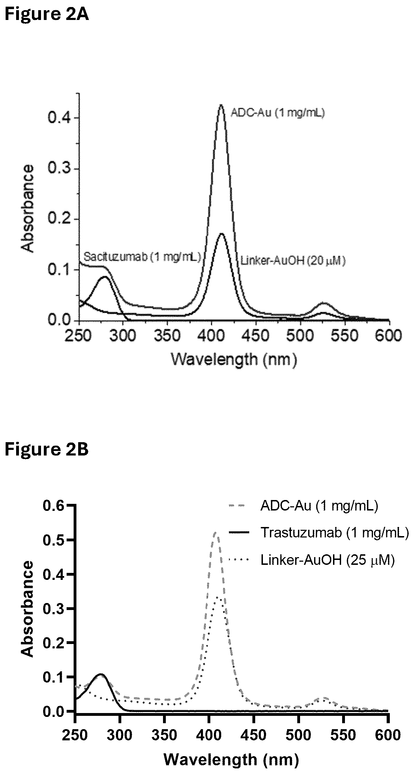

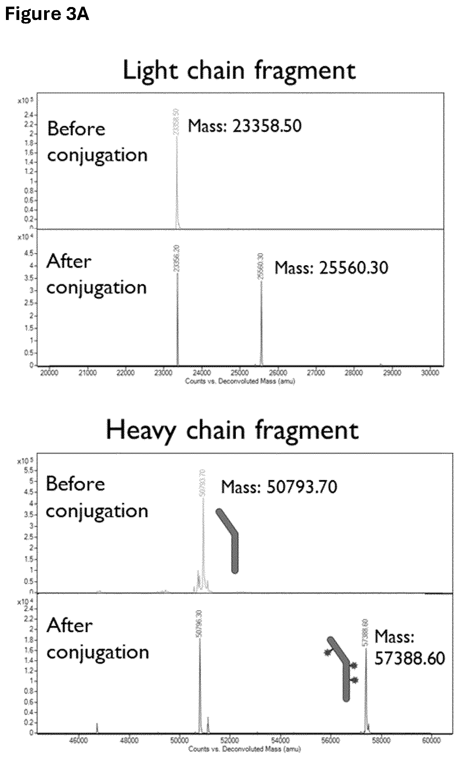

Reducing SDS-PAGE analysis indicated that some of the light chain and heavy chain fragments of Sacituzumab (mAb) was successfully conjugated with Linker-AuOH and migrated at a larger kDa (*see* Figure 1A). The UV-Vis absorption spectrum of ADC-Au also showed the absorption peaks of both AuOH and Sacituzumab (*see* Figure 2A). Finally, the deconvoluted mass spectra suggested that the drug-to-antibody ratio (DAR) was determined as 4.2 (*see* Figure 3A).

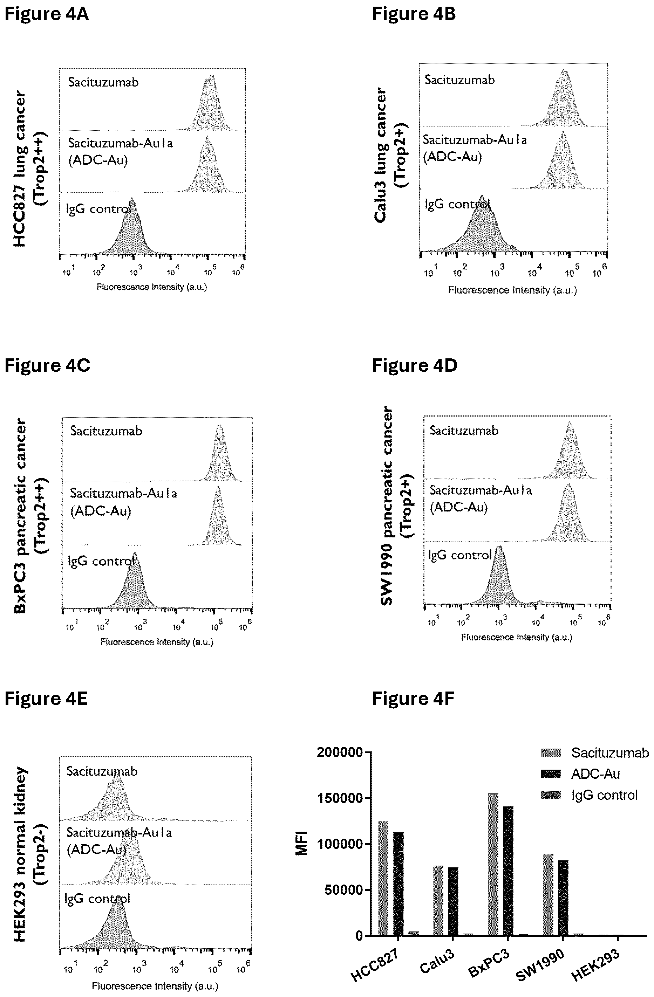

The TROP2-specific cellular binding and uptake of ADC-Au was then examined by flow cytometry and confocal microscopy using cell lines with different expression levels of TROP2 receptor. As shown in Figures 4A-4E, both ADC-Au and Sacituzumab showed significant specific-binding to the TROP2-overexpressed cancer cells, including HCC827 and Calu3 human lung cancer cells and BxPC3 and SW1990 human pancreatic cancer cells, but not to the TROP2-negative non-cancerous HEK293 human embryonic kidney cells. Figure 4F shows a bar graph of the quantified mean fluorescence intensity (MFI) of ADC-Au, which was similar to those of Sacituzumab, meaning that the binding affinity of the antibody was not significantly affected after conjugation with the gold(III) porphyrin.

At 4 °C, ADC-Au was mainly located on the cellular membrane of the TROP2-positive cancer cells, as determined by confocal microscopy imaging of Alexa 647-labelled ADC-Au in HCC827, BxPC3, SW1990, and HEK293 cells under different incubation conditions, where the cell nucleus was stained by Hoechst (images not shown). However, upon incubation at 37 °C, ADC-Au could be internalized efficiently and enter lysosome for degradation, which is the final compartment of receptor-mediated endocytosis, as determined by colocalization microscopy imaging of the ADC-Au with LysoTracker Green (images not shown).

Target specificity was demonstrated using a co-culture of TROP2-negative HEK293 and TROP2-positive HCC827 or BxPC3 cells, which were pre-stained with CellTrace Violet and Cell Tracker Green CMFDA, respectively, and incubated with Alexa 647-labelled ADC-Au at 37 °C for 4 h. Confocal microscopy images (not shown) shows that the ADC-Au could be selectively taken up by the TROP2-positive cancer cells by showing a red-dot fluorescence inside the green-coloured cancer cells. All these results demonstrated that ADC-Au was able to specifically target TROP2-overexpressed cancer cells.

The anti-proliferative activities of ADC-Au against HCC827, Calu3, BxPC3, SW1990 and SKBR3 were investigated and compared with Sacituzumab and AuOH (*see* Table 2 below). ADC-Au showed high cytotoxicity against TROP2-overexpressed cancer cells with the IC₅₀ values ranging from 20 to 170 nM, while the cell growth inhibition of the antibody was not obvious for all the cells.

**Table 2. IC₅₀ values of ADC, mAb and AuOH against different cell lines.**

| IC₅₀ (µM) | HCC827 | Calu3 | BxPC3 | SW1990 | SKBR3 |
|---|---|---|---|---|---|
| AuOH | 0.14 | 0.39 | 0.08 | 0.15 | 0.08 |
| ADC-Au | 0.04 | 0.17 | 0.02 | 0.09 | 0.03 |
| Sacituzumab | >2 | >2 | >2 | >2 | >2 |

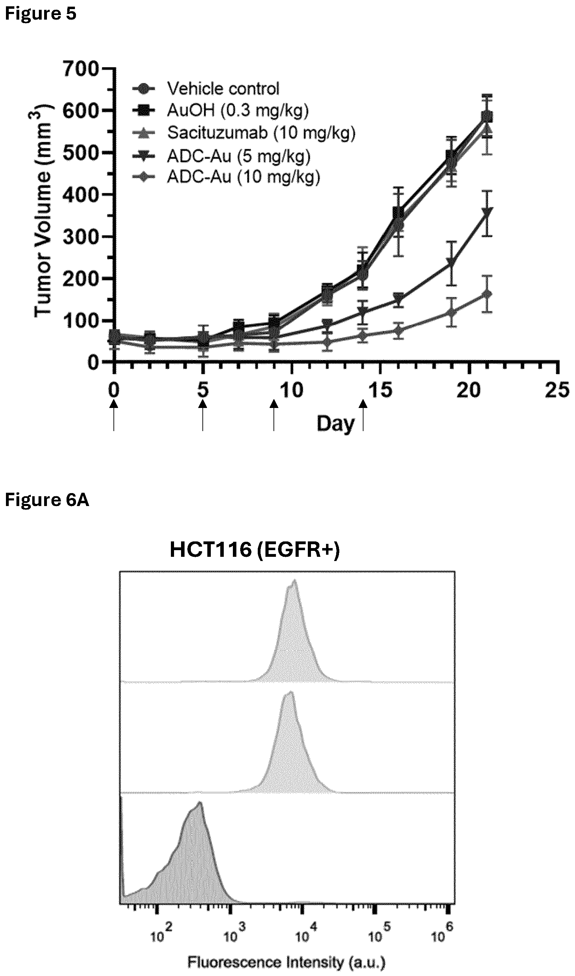

Lastly, the *in vivo* anti-tumor activity of ADC-Au was examined by using Trop2-positive HCC827 xenograft mice model, and the results were compared with the treatment of the native antibody (i.e. Sacituzumab) and the free drug AuOH (*see* Figure 5). After intravenous injection of ADC-Au at 10 mg/kg, the tumor growth was significantly inhibited with about 75% tumor size reduction compared to the vehicle control, while the treatment of AuOH (0.3 mg/kg) at the same equivalent gold content of ADC-Au showed negligible anti-tumor activity. These results demonstrated that conjugation with the tumor-targeting antibody could improve the in vivo anti-tumor activity, possibly due to the increased accumulation of cytotoxic payloads in the tumor.

### Example 2: Cetuximab-AuOH

### Preparation and Characterization of Cetuximab-AuOH:

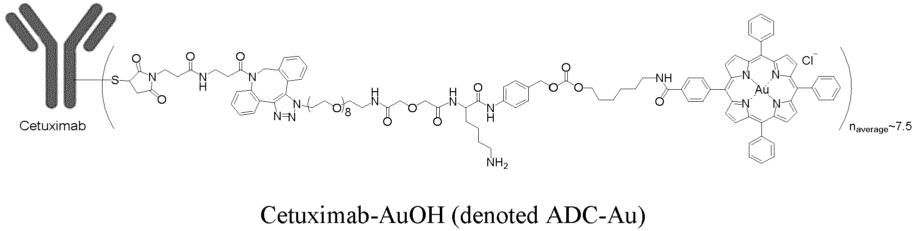

Cetuximab (MCE, 2.5 mg/mL) was dissolved in borate buffer (pH 8.0) and TCEP (10 equiv.) was added to the reaction mixture for 1.5 h. After the reduction of disulphide bond, Linker-AuOH (12 equiv.) dissolved in DMSO (8% v/v) was added and the reaction mixture was stirred for 2 h. The ADC-Au conjugate was purified by using Zeba^{™} Spin Desalting Column (Thermo Fisher, 7K MWCO) and washed with Amicon Ultra Centrifugal Filter (10 kDa MWCO) according to the manufacturer's protocol. The protein concentration was determined by the standard bicinchoninic acid (BCA) assay and the yield was determined to be 80%. The methods of characterization are described as above (in Example 1).

### In Vitro Anti-proliferative Activity and Flow Cytometric Study

The methods are described as above (*see* Example 1), except using Cetuximab and the corresponding ADC-Au.

### In Vivo Study

All animal experiments are conducted under the guidelines approved by the Committee on the Use of Live Animals in Teaching and Research of the University of Hong Kong. Female BALB/cAnN-nu (nude) mice (5-8 weeks old) are housed in the Laboratory Animal Unit, The University of Hong Kong. The mice are freely access to food and water. HT29 colorectal cancer cells (5 × 10⁶) in PBS (100 µL) are injected into the right flanks of each mouse subcutaneously. Once the tumor volumes reached about 50 mm³, the mice are divided into 5 groups (n = 5 for each group): (A) PBS vehicle control, (B) AuOH (0.9 mg/kg), (C) Cetuximab (15 mg/kg), (D) ADC-Au (10 mg/kg) and (E) ADC-Au (15 mg/kg). The mice are treated with intravenous administration of a total of four doses on day 0, 3, 6 and 9. The tumor size and body weight are monitored during the treatment. After 12 days, the mice are sacrificed.

### Results and Discussion:

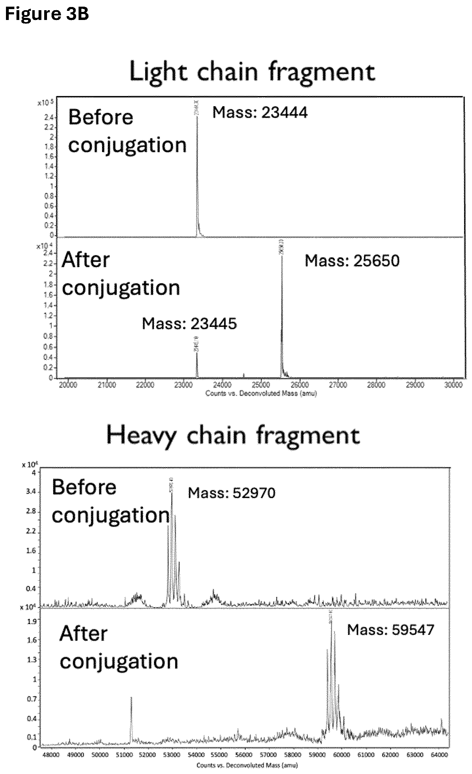

Similar to the first example of ADC, Cetuximab was successfully conjugated with Linker-AuOH with the DAR 7.5, which was confirmed by SDS-PAGE (*see* Figure 1B) and mass spectrometry (*see* Figure 3B).

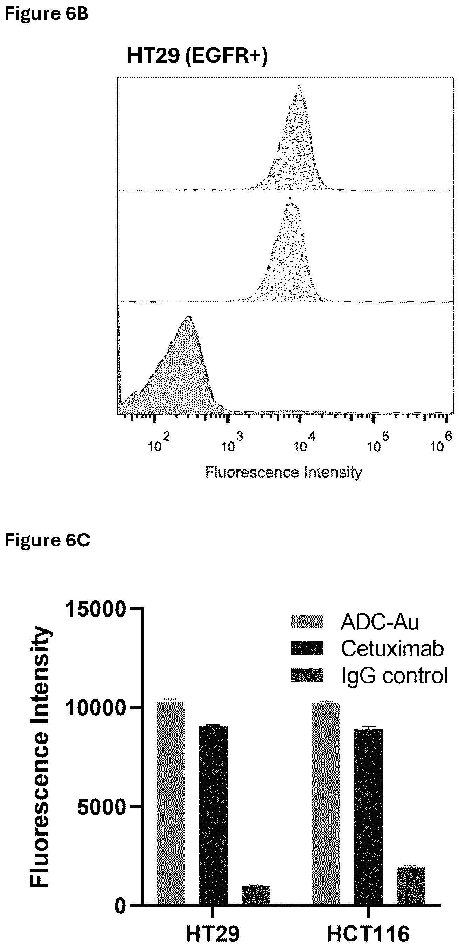

To determine the specific binding ability of Cetuximab against EGFR-overexpressed cancer cells, the ADC-Au, Cetuximab, and IgG control were treated with EGFR-positive HT29 and HCT116 cells and the cells were analyzed by flow cytometry. Figures 6A-6C showed that the ADC-Au demonstrated high binding affinity to these colorectal cancer cells, and the results are similar to those of Cetuximab, showing that the specific binding ability of the antibody is not affected after chemical modification.

The in vitro antiproliferative activity of ADC-Au was further investigated by NBB assay (Table 3 below). ADC-Au showed high cytotoxicity against EGFR-overexpressed HT29 and HCT116 cancer cells, of which the IC₅₀ value was determined to be 60 nM, while the cell growth inhibition of Cetuximab was not obvious for these cells, even up to 4 µM.

**Table 3. IC₅₀ values of ADC, mAb and AuOH against HT29 and HCT116 cell lines.**

| IC₅₀ (µM) | HT29 | HCT116 |
|---|---|---|
| AuOH | 0.12 | 0.16 |
| ADC-Au | 0.06 | 0.06 |
| Cetuximab | >4 | >4 |

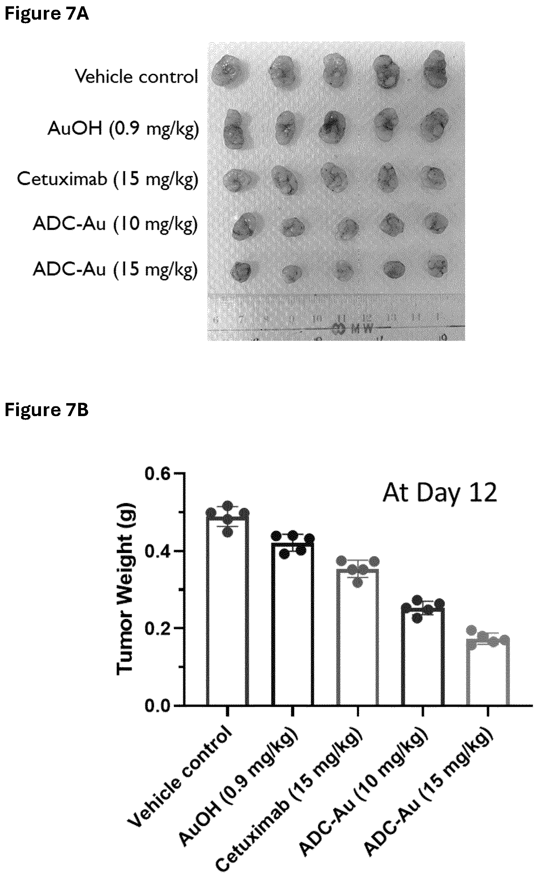

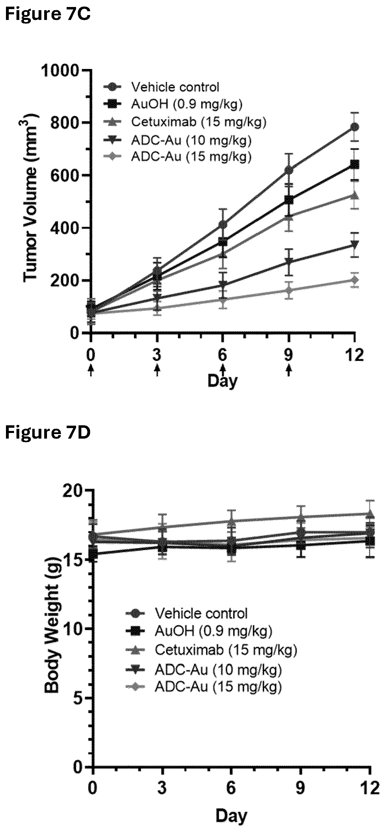

Lastly, the in vivo antitumor activity of the ADC-Au was examined with HT29 tumor-bearing mice. As shown in Figures 7A-7C, the tumor volume and weight were both significantly reduced after administration of four doses of ADC-Au at 15 mg/kg, while the treatment of Cetuximab at 15 mg/kg only demonstrated moderate antitumor effect. In addition, all these treatments did not show obvious side-effect on body weight (*see* Figure 7D).

### Example 3: Trastuzumab-AuOH

### Synthetic Methods and Characterization:

### Synthesis of Linker-AuOH:

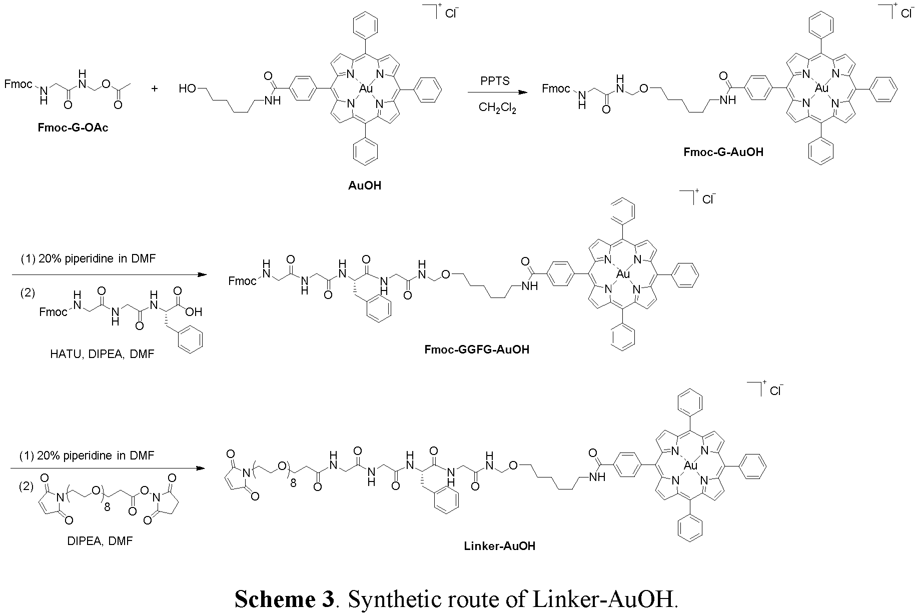

Fmoc-Gly-NH-CH2-acetyloxy (Fmoc-G-OAc) (7.4 mg, 20.2 µmol) was reacted with AuOH (10.0 mg, 10.1 µmol) in the presence of pyridinium p-toluenesulfonate (PPTS) (0.25 mg, 1.01 µmol) in dichloromethane CH₂Cl₂. The reaction mixture was refluxed at 40 °C overnight and the solvent was removed in vacuo. The crude product was purified by semi-preparative HPLC to afford Fmoc-G-AuOH (6.9 mg, 71%) after lyophilization. Subsequently, Fmoc was deprotected with 20% piperidine in DMF and condensing the resulting product with Fmoc-GGF-COOH in the presence of HATU and DIPEA in DMF could obtain the intermediate Fmoc-GGFG-AuOH after purification by size-exclusion chromatography on Bio-Beads S-X1 beads using CH₂Cl₂ as the eluent. Lastly, Fmoc was deprotected with 20% piperidine in DMF and the crude mixture was reacted with Mal-PEG8-NHS ester in the presence of DIPEA in DMF to afford the final product after purification by HPLC and lypophilization. HRMS (ESI): m/z for C₉₀H₉₉AuN₁₁O₁₇⁺ [M]⁺ calcd: 1802.6886; found 1802.6817.

### Preparation and Characterization of Trastuzumab-AuOH:

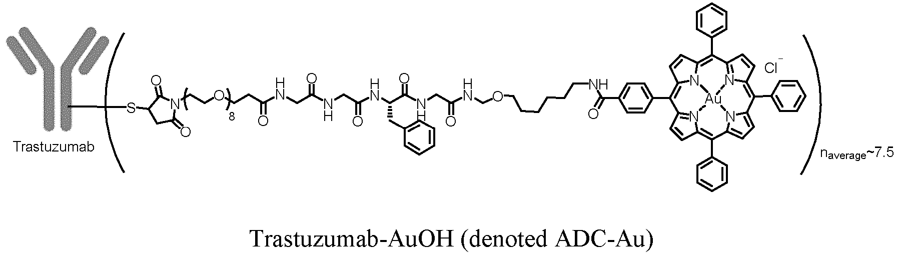

Trastuzumab (MCE, 2.5 mg/mL) was dissolved in borate buffer (pH 8.0) and TCEP (10 equiv.) was added to the reaction mixture for 1.5 h. After the reduction of disulphide bond, Linker-AuOH (12 equiv.) dissolved in DMSO (8% v/v) was added and the reaction mixture was stirred for 2 h. The ADC-Au conjugate was purified by using Zeba^{™} Spin Desalting Column (Thermo Fisher, 7K MWCO) and washed with Amicon Ultra Centrifugal Filter (10 kDa MWCO) according to the manufacturer's protocol. The protein concentration was determined by the standard bicinchoninic acid (BCA) assay and the yield was determined to be 85%. The methods of characterization are described as above (in Example 1).

### In Vitro Anti-proliferative Activity, Flow Cytometry and Confocal Microscopy Study

The methods are described as above (in Example 1), except using Trastuzumab and the corresponding ADC-Au.

### In vivo study

All animal experiments are conducted under the guidelines approved by the Committee on the Use of Live Animals in Teaching and Research of the University of Hong Kong. Female BALB/cAnN-nu (nude) mice (5-8 weeks old) are housed in the Laboratory Animal Unit, The University of Hong Kong. The mice are freely access to food and water. NCI-N87 gastric cancer cells (4 × 10⁶) in PBS (100 µL) are injected into the right flanks of each mouse subcutaneously. Once the tumor volumes reached about 100 mm³, the mice are divided into 4 groups (n = 6 for each group): (A) PBS vehicle control, (B) AuOH (0.5 mg/kg), (C) Trastuzumab (10 mg/kg), and (D) ADC-Au (10 mg/kg). The mice are treated with intravenous administration of a total of four doses on day 1, 5, 8 and 12. The tumor size and body weight are monitored during the treatment. After 18 days, the mice are sacrificed.

### Results and Discussion:

With reference to the design of the FDA-approved Trastuzumab Deruxtecan, AuOH was conjugated with Trastuzumab via a cathepsin B-cleavable tetrapeptide GGFG linker and a self-immolative amino methylene spacer. In addition, a PEG8 unit was installed to improve the hydrophilicity and solubility and reduce aggregation due to the hydrophobic porphyrin ligand. As shown in Scheme 3 above for the synthetic route for the preparation of Linker-AuOH precursor, AuOH was first treated with Fmoc-Gly-NH-CH2-acetyloxy in the presence of PPTS to install the self-immolative amino methylene spacer. After Fmoc deprotection, the peptide linker and the maleimide-appeneded PEG8 unit were further coupled on the AuOH sequentially through amide coupling to afford the final linker-payload conjugate (Linker-AuOH).

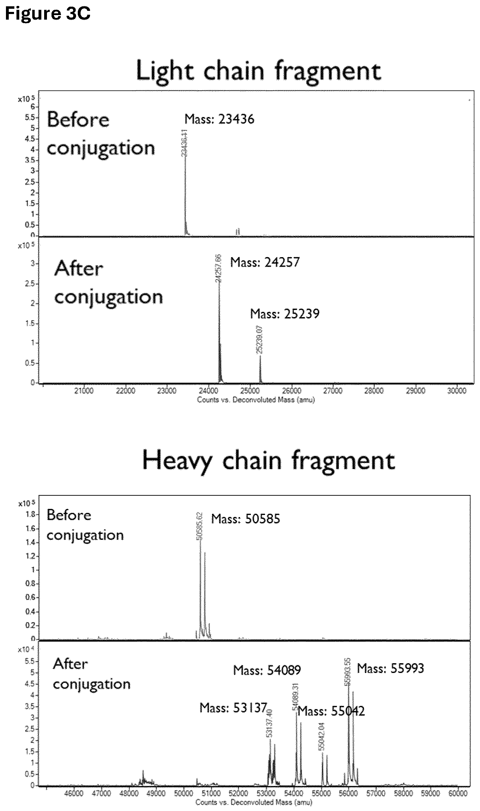

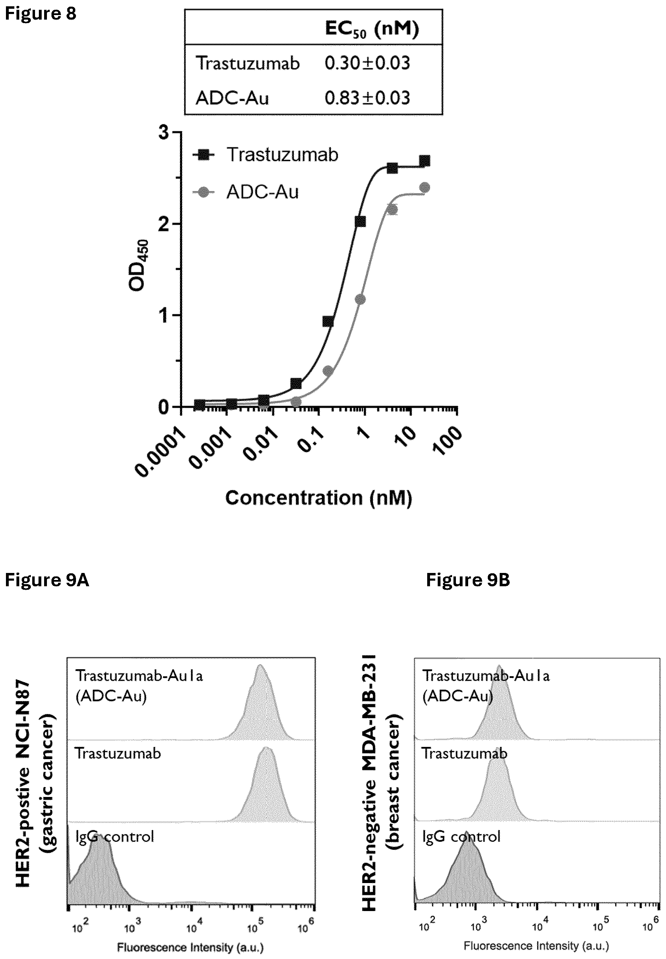

Next, the interchain disulfide bonds of Trastuzumab was partially reduced with TCEP·HCl and conjugated with Linker-AuOH via the conventional Michael addition in a borate buffered saline (pH 8.0). The resulting ADC Trastuzumab-AuOH (ADC-Au) was successfully obtained after purification by Zeba spin desalting column (7 kDa MWCO) and Amicon Ultra Centrifugal Filter (10 kDa MWCO), which was further characterized by reducing SDS-PAGE analysis (*see* Figure 1C), UV-Vis absorption spectroscopy (*see* Figure 2B), and high-resolution mass spectrometry (*see* Figure 3C). All the results indicated that gold porphyrin was successfully conjugated to Trastuzumab and the DAR was determined as 7.5. In addition, the ELISA study showed that the binding of ADC-Au to HER2 protein is not significantly different from those of Trastuzumab (*see* Figure 8), indicating that the chemical modification does not significantly affect the binding affinity of Trastuzumab.

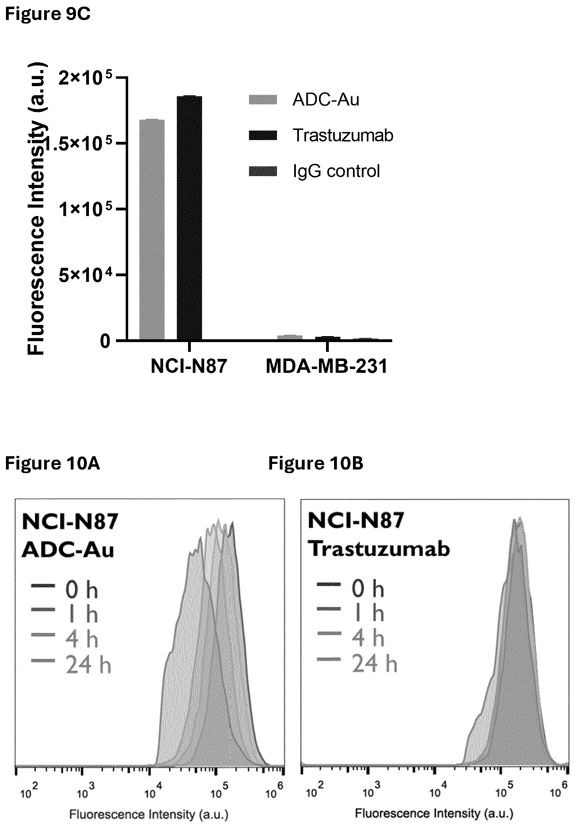

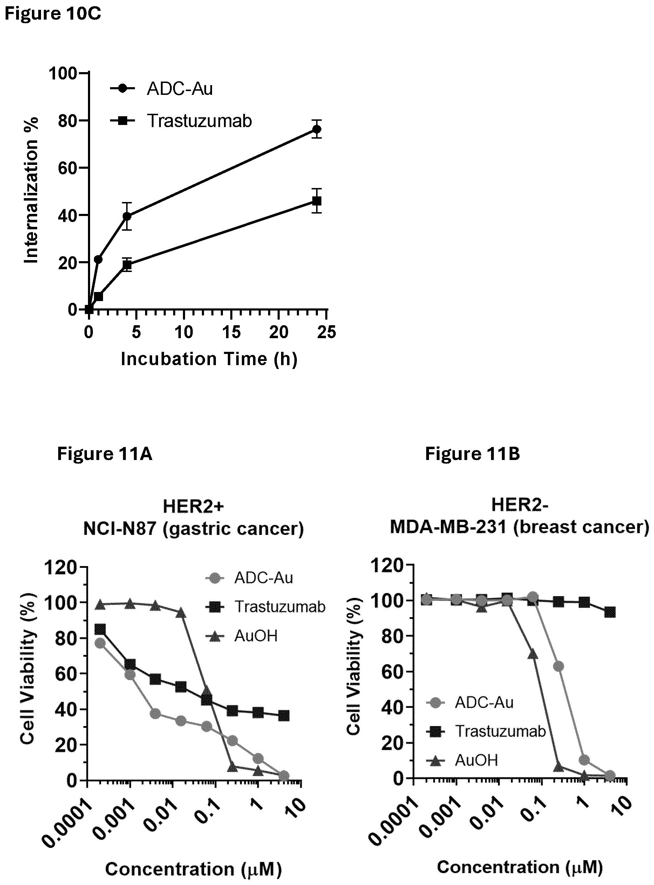

The HER2-specific cellular binding and uptake of ADC-Au was also examined by flow cytometry and confocal microscopy with the HER2-overexpressed NCI-N87 cells and the HER2-negative MDB-MB-231 cells. As shown in Figures 9A-9C, both ADC-Au and Trastuzumab showed similar and significant uptake to the NCI-N87 cells, but not to the MDB-MB-231 cells, demonstrating the high specificity of the antibody. Upon incubation at 37 °C for 24 h, the ADC-Au could internalize into HER2-positive cells through the receptors with about 80% internalization efficiency (*see* Figures 10A-11C). Interestingly, the internalization efficiency of ADC-Au was higher than that of the original antibody Trastuzumab, which could be attributed to the presence of PEG8 unit in the linker. Similarly, the confocal images showed that the ADC-Au was mainly located on the cellular membrane of the HER2-positive cancer cells at 4 °C (images not shown), but it could be internalized efficiently at 37 °C and enter lysosome for subsequent degradation (images not shown).

The anti-proliferative activities of ADC-Au against NCI-N87 and MDA-MB-231 cells were investigated and compared with Trastuzumab and AuOH (*see* Table 4 and Figures 11A and 11B). Trastuzumab showed cell growth inhibition for the HER2-overexpressed NCI-N87 cancer cells but not the HER2-negative MDA-MB-231 cells. After conjugation with gold porphyrin, the ADC-Au showed much higher cytotoxicity against NCI-N87 cancer cells with the IC₅₀ value of 2 nM, which is significantly more potent than that of the MDA-MB-231 cells.

**Table 4. IC₅₀ values of ADC, mAb and AuOH against different cell lines.**

| IC₅₀ | NCI-N87 | MDA-MB-231 |
|---|---|---|
| AuOH | 80 nM | 0.10 µM |
| ADC-Au | 2 nM | 0.28 µM |
| Trastuzumab | 20 nM | >4 µM |

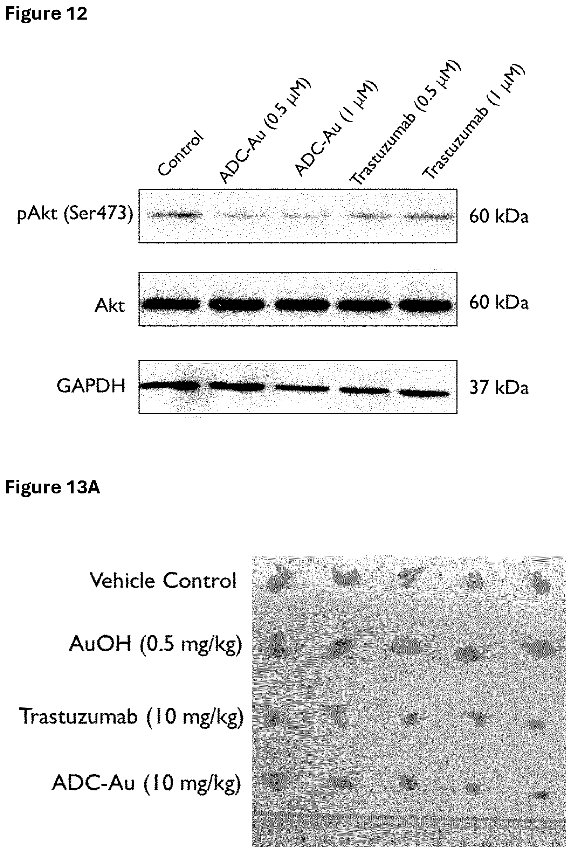

To further examine whether ADC-Au retains the same mechanism of action of Trastuzumab, western blot analysis was performed to study the intracellular pAkt (Ser473) in the NCI-N87 cells (*see* Figure 12). Both ADC-Au and Trastuzumab could induce the downregulation of the intracellular pAkt (Ser473) in the NCI-N87 cells after treatment for 24 hours, while the total Akt levels was not affected, indicating a reduction in the activation of the Akt signaling pathway and cell cycle arrest through AKT inhibition. These results demonstrated that ADC-Au could retain the functions of Trastuzumab after conjugation with gold porphyrin.

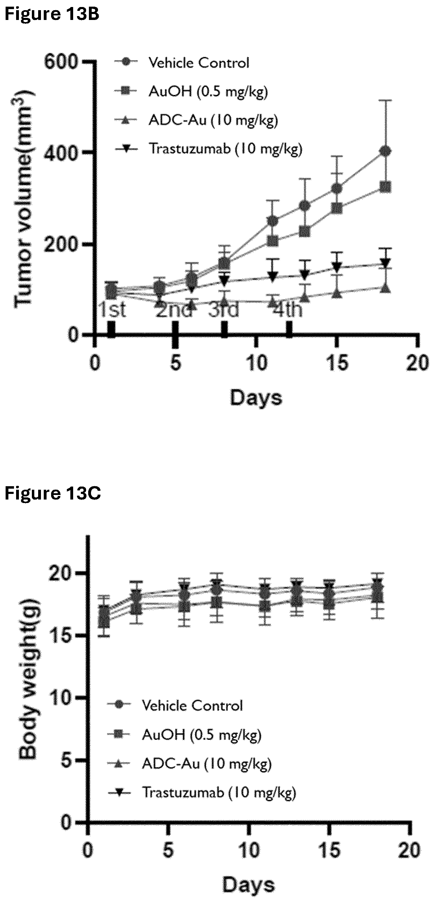

Lastly, the anti-tumor effect of the ADC-Au was investigated using NCI-N87 gastric cancer xenograft model and compared with those of AuOH and Trastuzumab. As shown in Figures 13A and 13B, the treatment of AuOH at 0.5 mg/kg, which is the equivalent dose of gold content in ADC-Au at 10 mg/kg, could only slightly attenuate the tumor growth. For the treatment of Trastuzumab (10 mg/kg), obvious tumor growth inhibition could be observed. However, the administration of four doses of ADC-Au at 10 mg/kg could lead to the most significant tumor growth regression, compared to the vehicle control. These results demonstrated that antibody could promote the tumor targeted delivery of the gold complex and hence lead to the promising in vivo anti-cancer activity. More importantly, all these treatments did not affect the body weight of mice (Figure 13C).

Unless defined otherwise, all technical and scientific terms used herein have the same meanings as commonly understood by one of skill in the art to which the disclosed invention belongs. Publications cited herein and the materials for which they are cited are specifically incorporated by reference.

Those skilled in the art will recognize, or be able to ascertain using no more than routine experimentation, many equivalents to the specific instances of the invention described herein. Such equivalents are intended to be encompassed by the following claims.

The invention may be defined by the following clauses:

### Clauses:

1. A metal-based antibody-drug conjugate comprising:
   a gold(III) porphyrin;
   a linker group; and
   an antibody; wherein the linker group covalently links the gold(III) porphyrin and the antibody.
2. The conjugate of clause 1, wherein the metal-based antibody-drug conjugate has a chemical structure according to Formula (I): 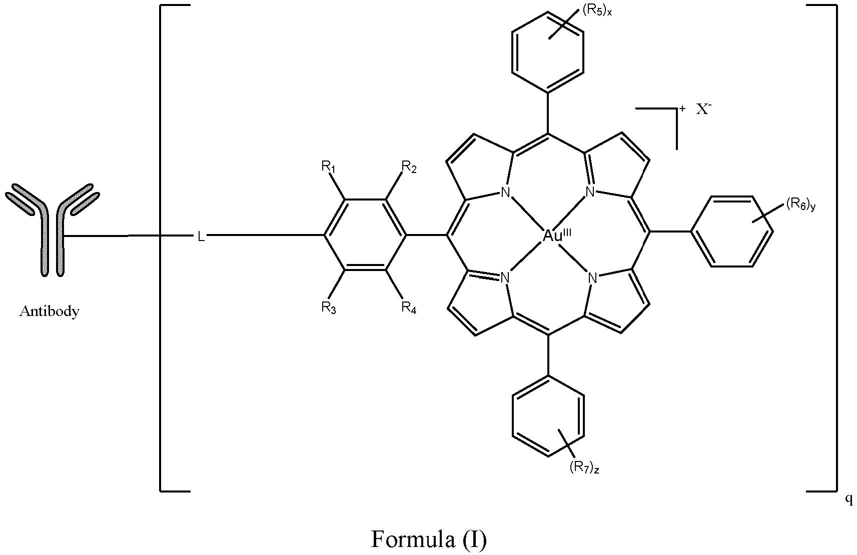
   wherein L is the linker group;
   wherein R₁, R₂, R₃, and R₄ are each independently selected from the group consisting of a hydrogen; halogen group; a C₂-C₅ alkyl group; alkenyl group; alkynyl group; cycloalkyl group; cycloalkenyl group; cycloalkynyl group; a hydroxyl group; an alkoxy group, such as methoxy, ethoxy, propoxy, or butoxy; an aryl group; a heteroaryl group; a benzyl group; an acyl group; an ester group; a carbonyl group; a carboxylate group; an amino group; an amide group; and a nitro group;
   wherein R₅, R₆, and R₇ are optional substituents and are each independently selected from the group consisting of a halogen group; a C₂-C₅ alkyl group; alkenyl group; alkynyl group; cycloalkyl group; cycloalkenyl group; cycloalkynyl group; a hydroxyl group; an alkoxy group; an aryl group; a heteroaryl group; a benzyl group; an acyl group; an ester group; a carbonyl group; a carboxylate group; an amino group; an amide group; and a nitro group, ; and x, y, and z are integer values independently selected from 0-5;
   wherein X⁻ is a pharmaceutically acceptable counterion; and
   wherein q is a value in a range of about 1 to 8.
3. The conjugate of clause 2, wherein R₁, R₂, R₃, and R₄ are each hydrogen and/or x, y, and z are each 0.
4. The conjugate of clause 2 or clause 3, wherein the linker group comprises an end-group, known as antibody-attachment moiety, such as a thiol-reactive Michael acceptor group or amine-reactive N-Hydroxysuccinimide, and/or the linker group comprises a spacer, and/or the linker group comprises one or more polyethylene (PEG) groups, and/or the linker group comprises a cleavable bond, optionally wherein the linker group comprises an acid cleavable bond and/or an enzyme-cleavable linker.
5. The conjugate of clause 4, wherein the acid cleavable bond is a carbonate bond and the enzyme-cleavable linker is a cathepsin B cleavable peptide (e.g. GGFG).
6. The conjugate of any one of clauses 2 to 5, wherein the linker L has a chemical structure according to a structure of Formula (II) shown below: 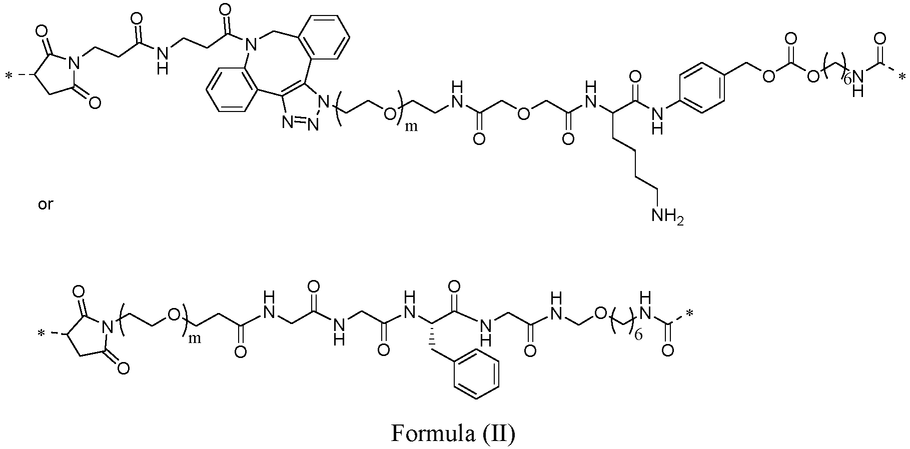 wherein m is an integer value in a range from 4 to 10.
7. The conjugate of any one of clauses 2 to 6, wherein the antibody is a monoclonal antibody; optionally wherein the monoclonal antibody is a TROP2-targeting, EGFR-targeting, or HER2-targeting monoclonal antibody.
8. The conjugate of clause 7, wherein the TROP2-targeting monoclonal antibody is Sacituzumab, the EGFR-targeting monoclonal antibody is Cetuximab, or the HER2-targeting monoclonal antibody is Trastuzumab.
9. The conjugate of any one of clauses 2 to 8, wherein the metal-based antibody-drug conjugate has one of the following structures: 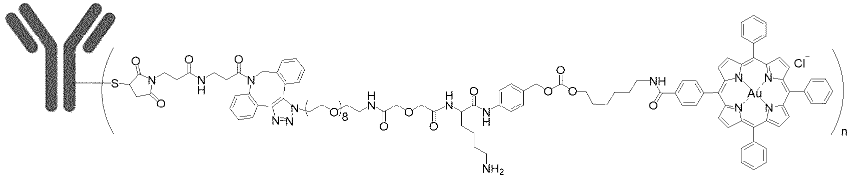 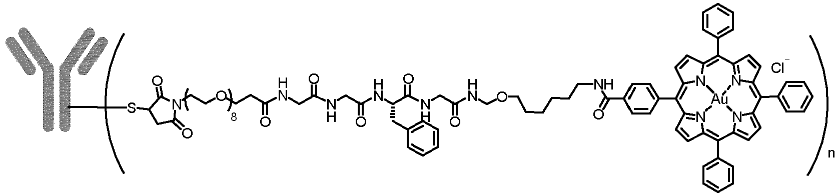 wherein n has a value of about 1 to 8 or is about 4 or 8.
10. A pharmaceutical composition comprising:
   a plurality of the metal-based antibody-drug conjugate of any one of clauses 1-9;
   one or more pharmaceutically acceptable carriers; and
   optionally one or more pharmaceutically acceptable excipients.
11. A method of targeted cancer therapy, the method comprising the steps of:
   (i) administering the pharmaceutical composition of clause 10 to a subject in need thereof.
12. The method of clause 11, wherein the subject has a cancer wherein the cancer cells thereof overexpress TROP2, EGFR, or HER2 receptor.
13. The method of clause 11 or clause 12, wherein the cancer is selected from the group consisting of breast, lung, liver, kidney, colorectal, gastric, ovarian, and pancreatic cancer.
14. The method of any one of clauses 11 to 13, where the plurality of the metal-based antibody-drug conjugate in the pharmaceutical composition provide a cytotoxicity against the TROP2, HER2, or EGFR-overexpressed cancer cells with an IC₅₀ value ranging from about 2 to 170 nM.
15. A method of making a metal-based antibody-drug conjugate comprising the steps of:
   (a) forming or providing a gold(III) porphyrin comprising a linker group; wherein the linker group comprises an end-group, known as antibody-attachment moiety, such as a thiol-reactive Michael acceptor group or amine-reactive N-Hydroxysuccinimide, and/or the linker group comprises a spacer, and/or the linker group comprises a cleavable bond; and
   (b) conjugating the gold(III) porphyrin to a monoclonal antibody to form the metal-based antibody-drug conjugate; wherein the monoclonal antibody comprises one or more thiol groups and at least one of the thiol groups undergoes a Michael addition with the Michael acceptor group of the linker group; or the monoclonal antibody comprises one or more amino groups and at least one of the amino groups undergoes a condensation reaction with the N-hydroxysuccinimide of the linker group.
16. The method of clause 15, wherein the gold(III) porphyrin has a structure according to Formula (III): 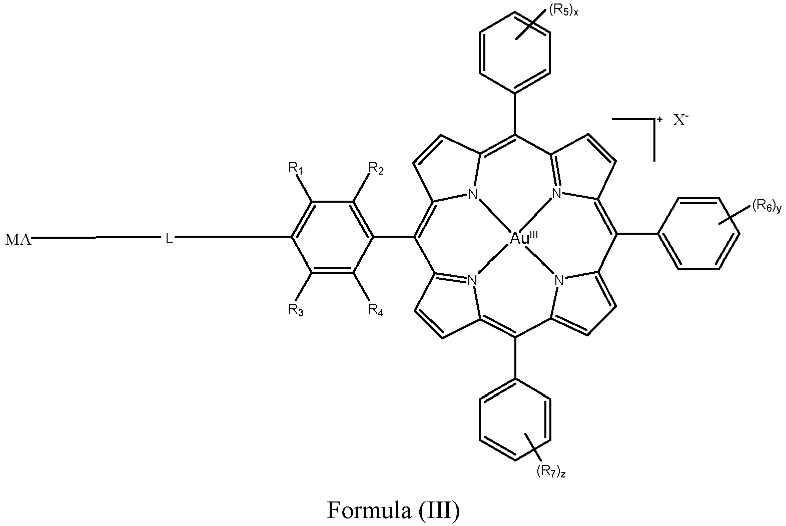
   wherein L is the linker group;
   wherein MA is the Michael acceptor group;
   wherein R₁, R₂, R₃, and R₄ are each independently selected from the group consisting of a hydrogen; halogen group; a C₂-C₅ alkyl group; alkynyl group; cycloalkyl group; cycloalkenyl group; cycloalkynyl group; a hydroxyl group; an alkoxy group, such as methoxy, ethoxy, propoxy, or butoxy; an aryl group; a heteroaryl group; a benzyl group; an acyl group; an ester group; a carbonyl group; a carboxylate group; an amino group; an amide group; and a nitro group;
   wherein R₅, R₆, and R₇ are optional substituents and are each independently selected from the group consisting of a halogen group; a C₂-C₅ alkyl group; alkynyl group; cycloalkyl group; cycloalkenyl group; cycloalkynyl group; a hydroxyl group; an alkoxy group; an aryl group; a heteroaryl group; a benzyl group; an acyl group; an ester group; a carbonyl group; a carboxylate group; an amino group; an amide group; and a nitro group, ; and x, y, and z are integer values independently selected from 0-5;
      and
   wherein X⁻ is a pharmaceutically acceptable counterion.
17. The method of clause 15 or clause 16, wherein the linker L has a chemical structure according to one of the following structures of Formula (IV): 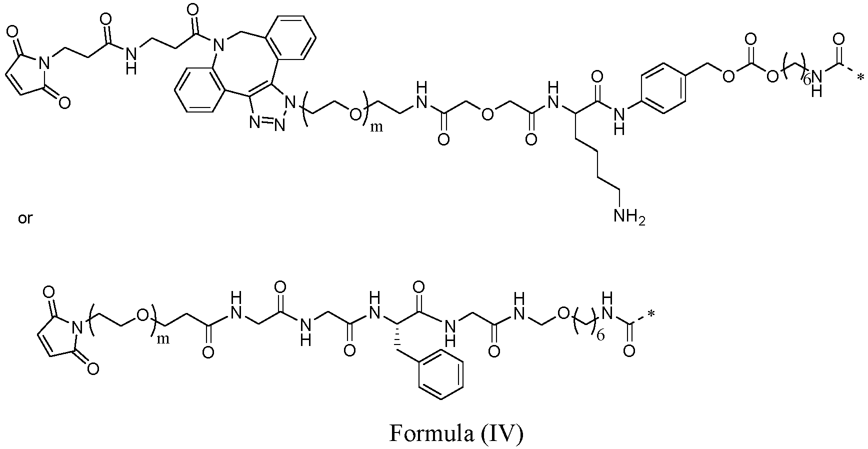 wherein m is an integer value in a range from 4 to 10.
18. The method of any one of clauses 15 to 17, wherein the monoclonal antibody targets tumor-associated receptors, such as TROP2, HER2, Nectin4, folate receptor alpha, or EGFR.
19. The method of clause 17, wherein the monoclonal antibody is a TROP2-targeting Sacituzumab, EGFR-targeting Cetuximab, or HER2-targeting Trastuzumab.
20. The method of any one of clauses 15 to 19, wherein the metal-based antibody-drug conjugate has one of the following structures: 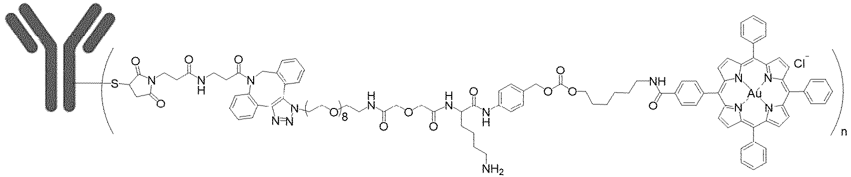 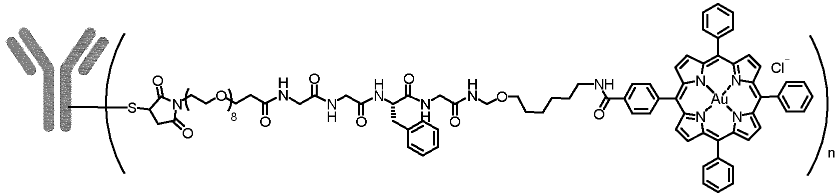 wherein n has a value of about 1 to 8 or is about 4 or 8.
21. A pharmaceutical composition according to clause 10 for use in a method of targeted cancer therapy, the method comprising administering the pharmaceutical composition to a subject in need thereof.
22. The pharmaceutical composition for use according to clause 21, wherein the subject has a cancer wherein the cancer cells thereof overexpress TROP2, EGFR, or HER2 receptor.
23. The pharmaceutical composition for use according to clause 21 or clause 22, wherein the cancer is selected from the group consisting of breast, lung, liver, kidney, colorectal, gastric, ovarian, and pancreatic cancer.
24. The pharmaceutical composition for use according to any one of clauses 21 to 23, where the plurality of the metal-based antibody-drug conjugate in the pharmaceutical composition provide a cytotoxicity against the TROP2, HER2, or EGFR-overexpressed cancer cells with an IC₅₀ value ranging from about 2 to 170 nM.

## Claims

1. A metal-based antibody-drug conjugate comprising:
a gold(III) porphyrin;
a linker group; and
an antibody;
wherein the linker group covalently links the gold(III) porphyrin and the antibody.

2. The conjugate of claim 1, wherein the metal-based antibody-drug conjugate has a chemical structure according to Formula (I): 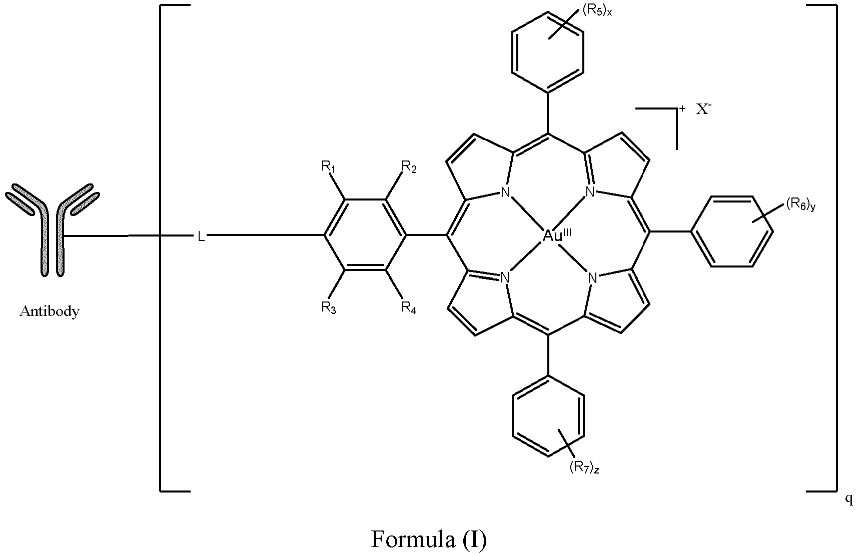
wherein L is the linker group;
wherein R₁, R₂, R₃, and R₄ are each independently selected from the group consisting of a hydrogen; halogen group; a C₂-C₅ alkyl group; alkenyl group; alkynyl group; cycloalkyl group; cycloalkenyl group; cycloalkynyl group; a hydroxyl group; an alkoxy group, such as methoxy, ethoxy, propoxy, or butoxy; an aryl group; a heteroaryl group; a benzyl group; an acyl group; an ester group; a carbonyl group; a carboxylate group; an amino group; an amide group; and a nitro group;
wherein R₅, R₆, and R₇ are optional substituents and are each independently selected from the group consisting of a halogen group; a C₂-C₅ alkyl group; alkenyl group; alkynyl group; cycloalkyl group; cycloalkenyl group; cycloalkynyl group; a hydroxyl group; an alkoxy group; an aryl group; a heteroaryl group; a benzyl group; an acyl group; an ester group; a carbonyl group; a carboxylate group; an amino group; an amide group; and a nitro group, ; and x, y, and z are integer values independently selected from 0-5;
wherein X⁻ is a pharmaceutically acceptable counterion; and
wherein q is a value in a range of about 1 to 8.

3. The conjugate of claim 2, wherein R₁, R₂, R₃, and R₄ are each hydrogen and/or x, y, and z are each 0.

4. The conjugate of claim 2 or claim 3, wherein the linker group comprises an end-group, known as antibody-attachment moiety, such as a thiol-reactive Michael acceptor group or amine-reactive N-Hydroxysuccinimide, and/or the linker group comprises a spacer, and/or the linker group comprises one or more polyethylene (PEG) groups, and/or the linker group comprises a cleavable bond,
optionally wherein the linker group comprises an acid cleavable bond and/or an enzyme-cleavable linker,
further optionally wherein the acid cleavable bond is a carbonate bond and the enzyme-cleavable linker is a cathepsin B cleavable peptide (e.g. GGFG).

5. The conjugate of any one of claims 2 to 4, wherein the linker L has a chemical structure according to a structure of Formula (II) shown below: 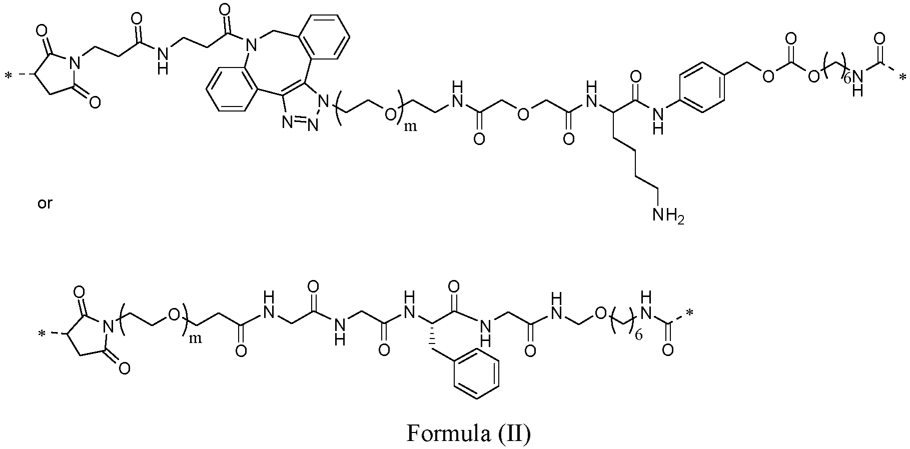 wherein m is an integer value in a range from 4 to 10.

6. The conjugate of any one of claims 2 to 5, wherein the antibody is a monoclonal antibody; optionally wherein the monoclonal antibody is a TROP2-targeting, EGFR-targeting, or HER2-targeting monoclonal antibody, further optionally wherein the TROP2-targeting monoclonal antibody is Sacituzumab, the EGFR-targeting monoclonal antibody is Cetuximab, or the HER2-targeting monoclonal antibody is Trastuzumab.

7. The conjugate of any one of claims 2 to 6, wherein the metal-based antibody-drug conjugate has one of the following structures: 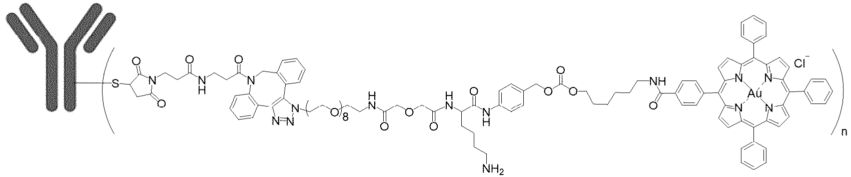 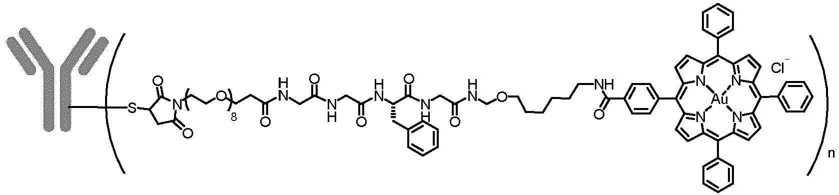 wherein n has a value of about 1 to 8 or is about 4 or 8.

8. A pharmaceutical composition comprising:
a plurality of the metal-based antibody-drug conjugate of any one of claims 1-7;
one or more pharmaceutically acceptable carriers; and
optionally one or more pharmaceutically acceptable excipients.

9. A pharmaceutical composition according to claim 8 for use in a method of targeted cancer therapy, the method comprising administering the pharmaceutical composition to a subject in need thereof.

10. The pharmaceutical composition for use according to claim 9, wherein:
(i) the subject has a cancer wherein the cancer cells thereof overexpress TROP2, EGFR, or HER2 receptor; and/or
(ii) the cancer is selected from the group consisting of breast, lung, liver, kidney, colorectal, gastric, ovarian, and pancreatic cancer.

11. The pharmaceutical composition for use according to claim 9 or 10, where the plurality of the metal-based antibody-drug conjugate in the pharmaceutical composition provide a cytotoxicity against the TROP2, HER2, or EGFR-overexpressed cancer cells with an IC₅₀ value ranging from about 2 to 170 nM.

12. A method of making a metal-based antibody-drug conjugate comprising the steps of:
(a) forming or providing a gold(III) porphyrin comprising a linker group; wherein the linker group comprises an end-group, known as antibody-attachment moiety, such as a thiol-reactive Michael acceptor group or amine-reactive N-Hydroxysuccinimide, and/or the linker group comprises a spacer, and/or the linker group comprises a cleavable bond; and
(b) conjugating the gold(III) porphyrin to a monoclonal antibody to form the metal-based antibody-drug conjugate; wherein the monoclonal antibody comprises one or more thiol groups and at least one of the thiol groups undergoes a Michael addition with the Michael acceptor group of the linker group; or the monoclonal antibody comprises one or more amino groups and at least one of the amino groups undergoes a condensation reaction with the N-hydroxysuccinimide of the linker group, optionally wherein the monoclonal antibody targets tumor-associated receptors, such as TROP2, HER2, Nectin4, folate receptor alpha, or EGFR, further optionally wherein monoclonal antibody is a TROP2-targeting Sacituzumab, EGFR-targeting Cetuximab, or HER2-targeting Trastuzumab.

13. The method of claim 12, wherein the gold(III) porphyrin has a structure according to Formula (III): 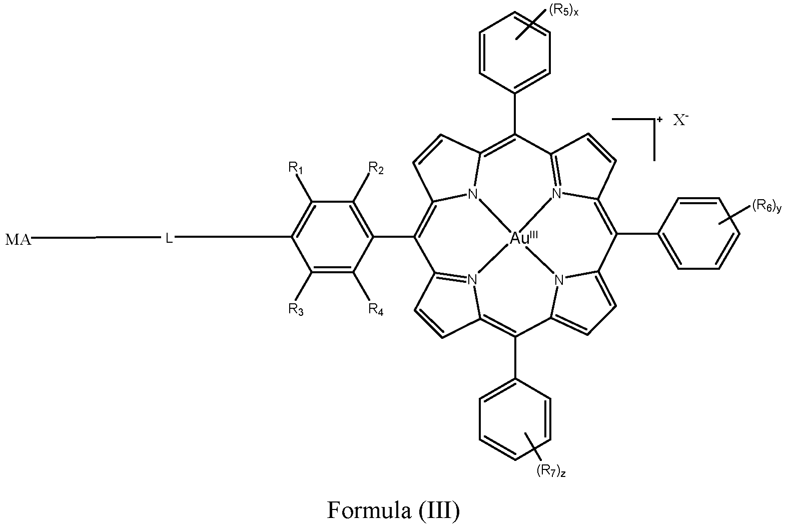
wherein L is the linker group;
wherein MA is the Michael acceptor group;
wherein R₁, R₂, R₃, and R₄ are each independently selected from the group consisting of a hydrogen; halogen group; a C₂-C₅ alkyl group; alkynyl group; cycloalkyl group; cycloalkenyl group; cycloalkynyl group; a hydroxyl group; an alkoxy group, such as methoxy, ethoxy, propoxy, or butoxy; an aryl group; a heteroaryl group; a benzyl group; an acyl group; an ester group; a carbonyl group; a carboxylate group; an amino group; an amide group; and a nitro group;
wherein R₅, R₆, and R₇ are optional substituents and are each independently selected from the group consisting of a halogen group; a C₂-C₅ alkyl group; alkynyl group; cycloalkyl group; cycloalkenyl group; cycloalkynyl group; a hydroxyl group; an alkoxy group; an aryl group; a heteroaryl group; a benzyl group; an acyl group; an ester group; a carbonyl group; a carboxylate group; an amino group; an amide group; and a nitro group, ; and x, y, and z are integer values independently selected from 0-5;
and
wherein X⁻ is a pharmaceutically acceptable counterion.

14. The method of claim 13, wherein the linker L has a chemical structure according to one of the following structures of Formula (IV): 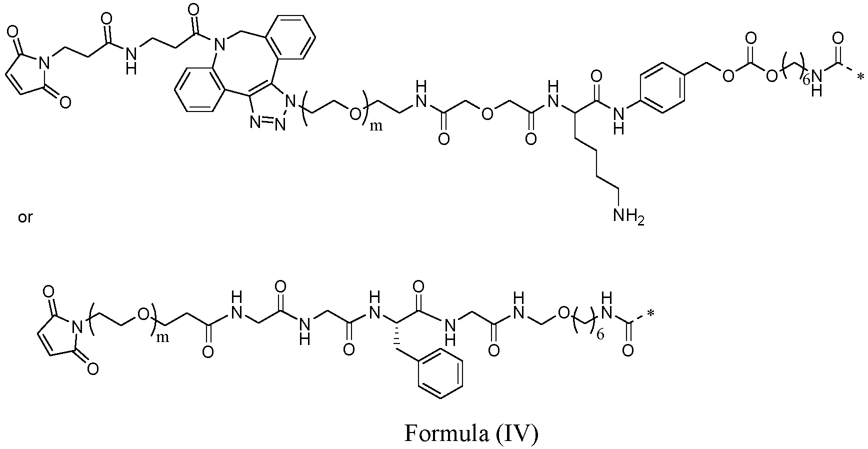 wherein m is an integer value in a range from 4 to 10.

15. The method of any one of claims 12 to 14, wherein the metal-based antibody-drug conjugate has one of the following structures: 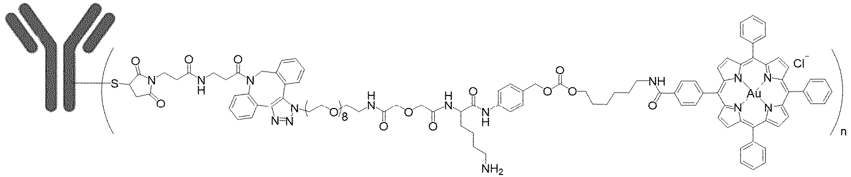 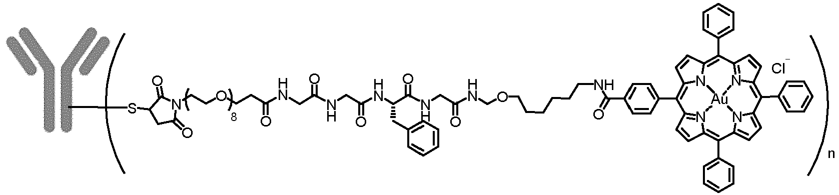 wherein n has a value of about 1 to 8 or is about 4 or 8.
